(19) Europäisches Patentamt
European Patent Office
Office européen des brevets

(11) EP 4 745 212 A1

(12) EUROPEAN PATENT APPLICATION

(43) Date of publication:
**20.05.2026 Bulletin 2026/21**

(21) Application number: **25214112.2**

(22) Date of filing: **06.11.2025**

(51) International Patent Classification (IPC):
***C09K 11/06*** $^{(2006.01)}$ ***H10K 50/16*** $^{(2023.01)}$

(52) Cooperative Patent Classification (CPC):
**C09K 11/06; H10K 50/16;** C09K 2211/1007; C09K 2211/104; C09K 2211/185

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **13.11.2024 KR 20240161050**
**10.03.2025 KR 20250030548**

(71) Applicant: **TOP RUN MATERIAL SOLUTION Co., Ltd.**
**Yangju-si, Gyeonggi-do 11410 (KR)**

(72) Inventors:
- **JANG, Joo Hee**
  **11410 Yangju-si (KR)**
- **KWON, Young Jae**
  **11410 Yangju-si (KR)**
- **LEE, A Reum**
  **11410 Yangju-si (KR)**
- **MOON, So Jeong**
  **11410 Yangju-si (KR)**
- **YOO, Jae Duk**
  **11410 Yangju-si (KR)**
- **YOO, , Seung Il**
  **11410 Yangju-si (KR)**
- **HYUN, A Reum**
  **11410 Yangju-si (KR)**

(74) Representative: **Habermann, Hruschka & Schnabel**
**Patentanwälte**
**Montgelasstraße 2**
**81679 München (DE)**

# (54) NEW COMPOUND AND ORGANIC LIGHT EMITTING DEVICE COMPRISING SAME

(57) A compound including 1,10-phenanthroline and quinoline-pyridine structural units is provided. The compound is suitable for use in an organic material layer such as a charge generation layer (CGL), an electron transport layer (ETL), or a hole blocking layer (HBL) of an organic light-emitting device. An organic light-emitting device including the compound is also provided.

EP 4 745 212 A1

## Description

## [TECHNICAL FIELD]

The present disclosure relates to a new compound and an organic light emitting device comprising the same, and more specifically, to a new compound capable of being applied to an organic material layer such as a charge generation layer (CGL), an electron transport layer (ETL), or a hole blocking layer (HBL) within an organic light emitting device by including a 1,10-phenanthroline and quinoline-pyridine structure, and an organic light emitting device comprising the same.

## [BACKGROUND]

The technology for an organic light emitting device, currently one of the most widely used flat-panel display devices, is rapidly developing.
Generally, an organic light emitting device is provided with an organic thin film layer, including an emission layer, formed between an anode (hole injection electrode) and a cathode (electron injection electrode), and has a principle that after holes injected from the anode and electrons injected from the cathode pair up in the emission layer, they emit light while disappearing.
More specifically, an organic light emitting device is formed by including an organic thin film layer formed between the anode and the cathode, the organic thin film layer may be formed by including a hole injection layer, a hole transport layer, an emission layer, an electron transport layer, and an electron injection layer, which are sequentially stacked on the anode, and holes injected from the anode and electrons injected from the cathode combine in the emission layer to form excitons so that they emit light while returning to a stable ground state after being excited to an unstable energy state (excited state).
Since color purity, color stability according to changes in current and voltage, ease of device manufacturing, etc. as well as high efficiency and long lifespan are important in the development of organic light emitting devices, research and development are in progress according to each method. The structure of organic light emitting devices may be broadly divided into a single layer light-emitting structure with one emission layer, a multilayer light-emitting structure with two or more emission layers, etc. Among these, a tandem structure of a multilayer light-emitting structure that stacks two or more emission layers is mainly adopted for organic light emitting devices with long lifespan.
Meanwhile, organic light emitting devices with such a tandem structure are provided with a charge generation layer (CGL) that is formed between the emission layers, which doubles the current efficiency generated in the emission layers and facilitates charge distribution. Such a CGL is a layer for generating charges, i.e., electrons and holes, and it doubles the current efficiency generated by the emission layers and facilitates charge distribution, thereby performing a role such as preventing an increase in operating voltage. Such a CGL is formed by including a P-type charge generation layer and an N-type charge generation layer.
As high-performance devices are increasingly demanded, the development of new structures capable of delivering superior performance is needed.

[Related Art Document]

[Patent Document]

Korean Patent Publication No. 10-2017-0093270 (Published on August 16, 2017)

## [SUMMARY]

An embodiment of the present disclosure provides a new compound that can be applied to organic material layers such as a charge generation layer (CGL), an electron transport layer (ETL), or a hole blocking layer (HBL) among organic material layers of an organic light emitting device, and an organic light emitting device which has improved performance, such as low voltage, high efficiency, and long lifespan by including the compound.
The above and additional tasks will be described in detail below.
The embodiment of the present disclosure is achieved by a new compound represented by Chemical Formula 1 below and an organic light emitting device including the same.

<Chemical Formula 1>

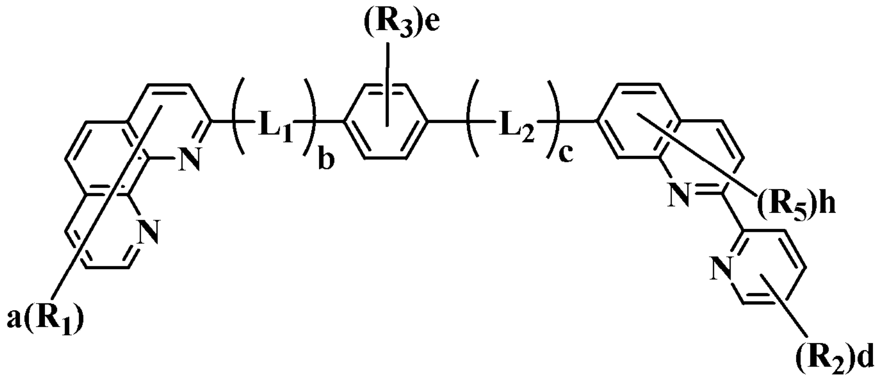

In Chemical Formula 1,

$R_1$ and $R_2$ are each independently selected from the group consisting of hydrogen, deuterium, a halogen group, a cyano group, a nitro group, a nitrile group, a substituted or unsubstituted $C_1$ to $C_{30}$ alkyl group, a substituted or unsubstituted $C_2$ to $C_{30}$ alkenyl group, a substituted or unsubstituted $C_2$ to $C_{30}$ alkynyl group, a substituted or unsubstituted $C_3$ to $C_{30}$ cycloalkyl group, a substituted or unsubstituted $C_2$ to $C_{30}$ heterocycloalkyl group, a substituted or unsubstituted $C_1$ to $C_{30}$ alkoxy group, a substituted or unsubstituted $C_1$ to $C_{30}$ sulfide group, a substituted or unsubstituted $C_6$ to $C_{30}$ aryl group, a substituted or unsubstituted $C_2$ to $C_{30}$ heteroaryl group, a substituted or unsubstituted $C_6$ to $C_{30}$ aryloxy group, a substituted or unsubstituted $C_2$ to $C_{30}$ heteroaryloxy group, a substituted or unsubstituted $C_1$ to $C_{30}$ thio group, a substituted or unsubstituted $C_1$ to $C_{30}$ amine group, a substituted or unsubstituted $C_1$ to $C_{30}$ silyl group, and a substituted or unsubstituted $C_1$ to $C_{30}$ phosphine oxide group,
$L_1$ and $L_2$ are each independently a direct bond or a substituted or unsubstituted $C_6$ to $C_{30}$ arylene group, and
$R_3$ and $R_5$ are each independently selected from the group consisting of hydrogen, deuterium, $-CD_3$, a halogen group, a cyano group, a nitro group, a nitrile group, a $C_1$ to $C_{30}$ alkyl group, and a $C_6$ to $C_{30}$ aryl group,
a is an integer from 0 to 7,
b and c are each independently an integer from 0 to 2,
d and e are each independently an integer from 0 to 4, and
h is an integer from 0 to 5.
In addition, in one embodiment,
the present disclosure provides an organic light emitting device including a first electrode, a second electrode, and at least one of organic material layers disposed between the first electrode and the second electrode, wherein one or more of the organic material layers contain the new compound.

In addition, in one embodiment,
the present disclosure provides an organic light emitting device in which the organic material layers containing the new compound are one or more of a charge generation layer (CGL), an electron transport layer (ETL), and a hole blocking layer (HBL).

The compound of the present disclosure can be applied to the electron transport layer, hole blocking layer, or charge generation layer of organic light emitting devices, and particularly, it can readily bind to dopants such as Li when applied to an N-type charge generation layer by including 1,10-phenanthroline, which possesses an unshared pair of electrons. Further, the unshared pair of electrons on nitrogens in quinoline-pyridine helps with more easily bonding with dopants, thereby making it desirable for them to be used also as a host for N-type charge generation layers using dopants such as not only Li but also Yb. Further, the compound of the present disclosure has effects of increasing device efficiency and the like as a result by having a low reorganization energy (RE) value of a certain level or lower and simultaneously satisfying a rate constant ratio of holes and electrons ($k_{et}(e)/k_{et}(h)$) of a certain level or higher at the same time. Further, the compound of the present disclosure has characteristics in which thermal stability is excellent through a structure which includes 1,4-phenylene as a linker.

The above and additional effects are described in detail below.

## [DETAILED DESCRIPTION]

In this specification, when a part is said to "include" a component, this means that other components may be further included rather than other components being excluded unless otherwise specifically stated.

In this specification, when a member is said to be located "on" another member, this includes not only cases where the member is in contact with the other member but also cases where another member exists between the two members.

In this specification, examples of substituents are described below, but are not limited thereto.

In this specification, the term "substitution" means that a hydrogen atom bonded to a carbon atom of a compound is replaced with another substituent. The position of substitution is possible without being particularly limited as long as it is a position where substitution is possible, and when substitution is made with two or more substituents, the substituents may be the same or different.

In this specification, the term "substituted or unsubstituted" means that a compound is substituted with one or more substituents selected from the group consisting of deuterium, a halogen group, a cyano group, a nitro group, a nitrile group, an alkyl group, an alkenyl group, an alkynyl group, a cycloalkyl group, a heterocycloalkyl group, an alkoxy group, a sulfide group, an aryloxy group, a heteroaryloxy group, a thio group, an amine group, a silyl group, a phosphine oxide group, an aryl group, and a heteroaryl group, is substituted with a substituent in which two or more substituents among the substituents selected from the above group are connected, or does not have any substituent, and the selected substituents may or may not form a ring by combining with each other. The substituent in which two or more substituents are connected may be a biphenyl group as an example. That is, the biphenyl group may correspond to an aryl group, simultaneously correspond to a substituent in which two phenyl groups are connected, and also simultaneously correspond to an aryl group in which one phenyl group is substituted.

In this specification, the alkyl group may be a straight or branched chain having 1 to 60 carbon atoms, and specific examples thereof include methyl, ethyl, n-propyl, isopropyl, n-butyl, isobutyl, tert-butyl, sec-butyl, 1-methylbutyl, 1-ethylbutyl, n-pentyl, isopentyl, neopentyl, tert-pentyl, n-hexyl, 1-methylpentyl, 2-methylpentyl, 3,3-dimethylbutyl, 2-ethylbutyl, n-heptyl, 1-methylhexyl, cyclopentylmethyl, cyclohexylmethyl, n-octyl, tert-octyl, 1-methylheptyl, 2-ethylhexyl, 2-propylpentyl, n-nonyl, 2,2-dimethylheptyl, 1-ethylpropyl, 1,1-dimethylpropyl, isohexyl, 2-methylpentyl, 4-methylhexyl, 5-methylhexyl, etc., but are not limited thereto. Specifically, the number of carbon atoms in the alkyl group may be 1 to 30, and more specifically, 1 to 20.

In this specification, the alkenyl group may be a straight or branched chain having 2 to 60 carbon atoms, and specific examples thereof include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, and a styrenyl group, but are not limited thereto. Specifically, the alkenyl group may have 2 to 30 carbon atoms, and more specifically, 2 to 20 carbon atoms.

In this specification, the alkynyl group may be a straight or branched chain having 2 to 60 carbon atoms. Specifically, the alkynyl group may have 2 to 30 carbon atoms, and more specifically, 2 to 20 carbon atoms.

In this specification, the alkoxy group may be a straight chain, branched chain or cyclic chain having 1 to 60 carbon atoms, and specific examples thereof include methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, etc., but are not limited thereto. Specifically, the alkoxy group may have 1 to 30 carbon atoms, and more specifically, 1 to 20 carbon atoms.

In this specification, the cycloalkyl group may be a monocyclic or polycyclic cycloalkyl group having 3 to 60 carbon atoms, and specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, etc., but are not limited thereto. Specifically, the cycloalkyl group may have 3 to 30 carbon atoms, and more specifically, 3 to 20 carbon atoms.

In this specification, the heterocycloalkyl group is a group that contains one or more non-carbon atoms, i.e., heteroatoms, and specifically, may be a cycloalkyl group that contains one or more heteroatoms selected from the group consisting of O, N, S, Se, etc., and may be a monocyclic or polycyclic heterocycloalkyl group having 2 to 60 carbon atoms. Specifically, the heterocycloalkyl group may have 2 to 30 carbon atoms, and more specifically, 2 to 20 carbon atoms.

In this specification, the sulfide group includes S and may have 1 to 60 carbon atoms. Specific examples thereof include an alkyl sulfide group such as a dimethyl sulfide group, an aryl sulfide group such as diphenyl sulfide, a heteroaryl sulfide group in which a heteroaryl group is substituted, etc., but are not limited thereto. Specifically, the sulfide group may have 1 to 30 carbon atoms, and more specifically, 1 to 20 carbon atoms.

In this specification, the aryloxy group includes O and may be a substituent in which the O atom is directly connected as a radical, the aryloxy group may have 6 to 60 carbon atoms, and specific examples thereof include a phenoxy group, a naphthoxy group, a biphenoxy group, or the like as an oxy group in which an aryl group is substituted, but are not limited thereto. The heteroaryloxy group is an oxy group in which a heteroaryl group is substituted and may have 2 to 60 carbon atoms. Specifically, the aryloxy group may have 6 to 30 carbon atoms, and more specifically, 6 to 20 carbon atoms.

In this specification, the thio group includes S and may have 1 to 60 carbon atoms. Specific examples thereof

include an alkylthio group such as a methylthio group, an ethylthio group, a butylthio group, a pentylthio group, or a hexylthio group, an arylthio group such as a phenylthio group or a naphthylthio group, a heteroarylthio group substituted with a heteroaryl group, etc., but are not limited thereto. Specifically, the thio group may have 1 to 30 carbon atoms, and more specifically, 1 to 20 carbon atoms.

In this specification, the silyl group may be a substituent which contains Si, and in which the Si atom is directly connected as a radical, and may have 1 to 60 carbon atoms. Specific examples thereof include an alkylsilyl group such as a trimethylsilyl group, a triethylsilyl group, a t-butyldimethylsilyl group, a vinyldimethylsilyl group, or a propyldimethylsilyl group, an arylsilyl group such as a triphenylsilyl group, a diphenylsilyl group, or a phenylsilyl group, a heteroarylsilyl group substituted with a heteroaryl group, etc., but are not limited thereto. Specifically, the silyl group may have 1 to 30 carbon atoms, and more specifically, 1 to 20 carbon atoms.

In this specification, the phosphine oxide group includes P=O and may have 1 to 60 carbon atoms, and specific examples thereof include an alkylphosphine oxide group such as a dimethylphosphine oxide group, an arylphosphine oxide group such as a diphenylphosphine oxide group or a dinaphthylphosphine oxide group, a heteroarylphosphine oxide group in which a heteroaryl group is substituted, etc., but are not limited thereto. Specifically, the phosphine oxide group may have 1 to 30 carbon atoms, and more specifically, 1 to 20 carbon atoms.

In this specification, the aryl group may be a monocyclic or polycyclic aryl group having 6 to 60 carbon atoms. In the case of the monocyclic aryl group, specific examples of include a phenyl group, a biphenyl group, a terphenyl group, etc., but are not limited thereto. In the case of the polycyclic aryl group, specific examples thereof include a naphthyl group, an anthracenyl group, a phenanthrenyl group, a triphenylenyl group, a pyrenyl group, a fluorenyl group, etc., but are not limited thereto. Specifically, the aryl group may have 6 to 50 carbon atoms, and more specifically, 6 to 30 carbon atoms.

In this specification, the heteroaryl group contains one or more non-carbon atoms, i.e., heteroatoms, and may specifically include one or more heteroatoms selected from the group consisting of O, N, S, Se, etc., and may be a monocyclic or polycyclic heteroaryl group having 2 to 60 carbon atoms. Specific examples thereof include a thiophenyl group, a furanyl group, a pyrrolyl group, an imidazolyl group, a thiazolyl group, an oxazolyl group, an oxadiazolyl group, a pyridinyl group, a bipyridinyl group, a pyrimidinyl group, a triazinyl group, a triazolyl group, an acridinyl group, a pyridazinyl group, a pyrazinyl group, a quinolinyl group, a quinazolyl group, a quinoxalinyl group, a phthalazinyl group, a pyridopyrimidinyl group, a pyridopyrazinyl group, a pyrazinopyrazinyl group, a pyridoindolyl group, a benzothienopyrimidyl group, an indenocarbazolyl group, an isoquinolinyl group, an indolyl group, a carbazolyl group, a benzoxazolyl group, a benzimidazolyl group, a benzothiazolyl group, a benzocarbazolyl group, a benzothiophenyl group, a dibenzothiophenyl group, a benzofuranyl group, a phenanthridinyl group, a phenanthrolinyl group, an isoxazolyl group, a thiadiazolyl group, a phenothiazinyl group, a dibenzofuranyl group, etc., but are not limited thereto. Specifically, the heteroaryl group may have 2 to 50 carbon atoms, and more specifically, 2 to 30 carbon atoms.

In this specification, the amine group may be selected from the group consisting of $-NH_2$, an alkylamine group, an N-alkylarylamine group, an arylamine group, an N-arylheteroarylamine group, an N-alkylheteroarylamine group, and a heteroarylamine group.

In this specification, the arylene group refers to a divalent aryl group having two bonding positions at the aryl group, and the heteroarylene group also refers to a divalent heteroaryl group having two bonding positions at the heteroaryl group. The descriptions of the aryl group and heteroaryl group described above may be applied to these except that each of them is a divalent group.

In the chemical formulas or structural formulas in this specification, * or 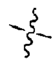 indicates a bonding position.

In this specification, identical symbols within one chemical formula or structural formula may be the same or different.

When a range such as "$C_2$ to $C_{50}$", "0 to 7", or the like is described in this specification, the range may be reduced to various ranges within the described range although there is no a special description and is deemed to be described in this specification. For example, $C_2$ to $C_{50}$ is deemed to describe various reduced ranges such as $C_2$ to $C_{50}$, $C_5$ to $C_{50}$, $C_6$ to $C_{30}$, $C_6$ to $C_{20}$, $C_6$ to $C_{15}$, $C_6$ to $C_{10}$, and $C_{12}$ to $C_{30}$ together with $C_2$ to $C_{50}$. Therefore, the numerical ranges described in this specification may be subject to reduction or correction in the future.

Throughout this specification, the term "interaction with a dopant" may refer to the coordination of an organic compound with a dopant, such as an alkali metal, alkaline earth metal, rare earth metal, lanthanum metal, or the like, within an N-type charge generation layer, and according to one embodiment, this may include the formation of a gap state by coordination of the organic compound and the dopant.

Meanwhile, various embodiments of the present disclosure may be combined with any other embodiments unless explicitly stated otherwise. Hereinafter, embodiments of the present disclosure and the effects according thereto will be described.

Hereinafter, a new compound of the present disclosure and an organic light emitting device including the same will be described.

The new compound of the present disclosure may be represented by Chemical Formula 1 below.

<Chemical Formula 1>

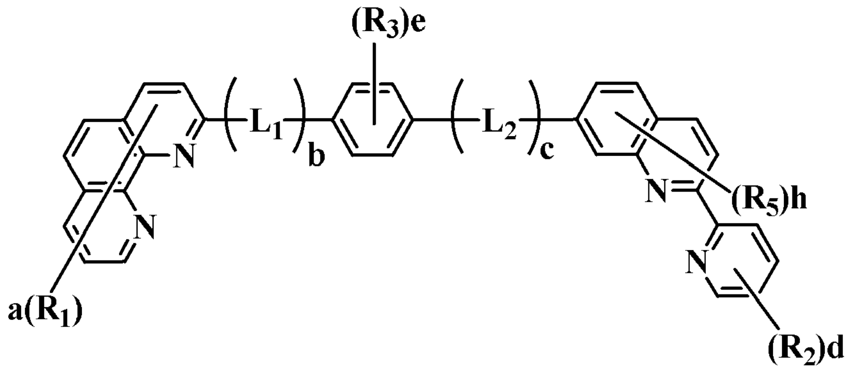

In Chemical Formula 1,

$R_1$ and $R_2$ are each independently selected from the group consisting of hydrogen, deuterium, a halogen group, a cyano group, a nitro group, a nitrile group, a substituted or unsubstituted $C_1$ to $C_{30}$ alkyl group, a substituted or unsubstituted $C_2$ to $C_{30}$ alkenyl group, a substituted or unsubstituted $C_2$ to $C_{30}$ alkynyl group, a substituted or unsubstituted $C_3$ to $C_{30}$ cycloalkyl group, a substituted or unsubstituted $C_2$ to $C_{30}$ heterocycloalkyl group, a substituted or unsubstituted $C_1$ to $C_{30}$ alkoxy group, a substituted or unsubstituted $C_1$ to $C_{30}$ sulfide group, a substituted or unsubstituted $C_6$ to $C_{30}$ aryl group, a substituted or unsubstituted $C_2$ to $C_{30}$ heteroaryl group, a substituted or unsubstituted $C_6$ to $C_{30}$ aryloxy group, a substituted or unsubstituted $C_2$ to $C_{30}$ heteroaryloxy group, a substituted or unsubstituted $C_1$ to $C_{30}$ thio group, a substituted or unsubstituted $C_1$ to $C_{30}$ amine group, a substituted or unsubstituted $C_1$ to $C_{30}$ silyl group, and a substituted or unsubstituted $C_1$ to $C_{30}$ phosphine oxide group,
$L_1$ and $L_2$ are each independently a direct bond or a substituted or unsubstituted $C_6$ to $C_{30}$ arylene group, and
$R_3$ and $R_5$ are each independently selected from the group consisting of hydrogen, deuterium, $-CD_3$, a halogen group, a cyano group, a nitro group, a nitrile group, a $C_1$ to $C_{30}$ alkyl group, and a $C_6$ to $C_{30}$ aryl group,
a is an integer from 0 to 7,
b and c are each independently an integer from 0 to 2,
d and e are each independently an integer from 0 to 4, and
h is an integer from 0 to 5.

According to one embodiment of the present disclosure, $R_1$ and $R_2$ may be each independently selected from the group consisting of hydrogen, deuterium, a cyano group, a methyl group, $-CD_3$, a phenyl group, a biphenyl group, a naphthyl group, a pyridine group, a pyrimidine group, a quinoline group, an isoquinoline group, a quinazoline group, and a naphthyridine group, and each of these may be further substituted with one or more of deuterium, a cyano group, a methyl group, $-CD_3$, a phenyl group, a naphthyl group, and a pyridine group. Specifically, $R_1$ may each be selected from hydrogen, deuterium, a phenyl group, and a pyridine group, and $R_2$ may each be selected from the group consisting of hydrogen, deuterium, a cyano group, a phenyl group, and a pyridine group. These $R_1$ and $R_2$ may be the same as or different from each other, and $R_1$s or $R_2$s may also be the same as or different from each other.

According to one embodiment of the present disclosure, $R_3$ and $R_5$ may be each independently selected from the group consisting of hydrogen, deuterium, a cyano group, a methyl group, $-CD_3$, a phenyl group, a biphenyl group, and a naphthyl group, and each of these may be further substituted with one or more of deuterium, a cyano group, a methyl group, $-CD_3$, a phenyl group, and a naphthyl group. Specifically, $R_3$ and $R_5$ may each be selected from the group consisting of hydrogen, deuterium, a cyano group, and a phenyl group, and more specifically, may be hydrogen or deuterium. These $R_3$ and $R_5$ may be the same as or different from each other, and $R_3$s or $R_5$s may also be the same as or different from each other.

According to one embodiment of the present disclosure, $L_1$ and $L_2$ may be each independently a direct bond or a $C_6$ to $C_{30}$ arylene group substituted or unsubstituted with one or more substituents selected from the group consisting of deuterium, $-CD_3$, a halogen group, a cyano group, a nitro group, a nitrile group, a $C_1$ to $C_{30}$ alkyl group, and a $C_6$ to $C_{30}$ aryl group. Specifically, $L_1$ and $L_2$ may be each independently a direct bond or a $C_6$ to $C_{30}$ arylene group substituted or unsubstituted with one or more substituents selected from deuterium, $-CD_3$, a cyano group, a methyl group, a phenyl group, and a naphthyl group. More specifically, $L_1$ and $L_2$ may be each independently a direct bond or a $C_6$ to $C_{20}$ arylene group substituted or unsubstituted with one or more substituents selected from deuterium, a cyano group, and a phenyl group, wherein the $C_6$ to $C_{20}$ arylene group may be selected from the group consisting of a phenylene group, a biphenylene group, and a naphthylene group.

According to one embodiment of the present disclosure, $L_1$ and $L_2$ may be each independently a direct bond or

may be selected from the structural formulas L-1 to L-13 below. $L_1$ and $L_2$ may be the same as or different from each other, and $L_1$s or $L_2$s may also be the same as or different from each other. The structural formulas L-1 to L-13 below are not fixed in terms of up/down or left/right and can be changed depending on all possible cases.

L-1
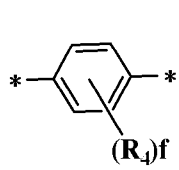
L-2
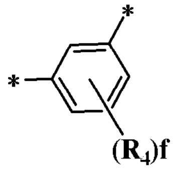
L-3
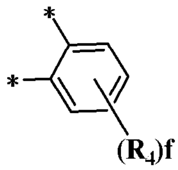

L-4
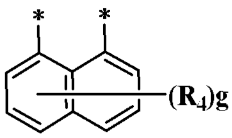
L-5
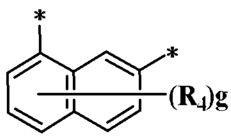
L-6
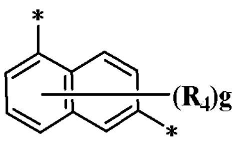
L-7
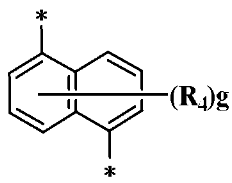
L-8
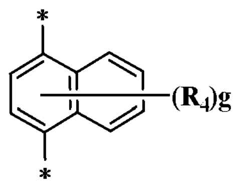

L-9
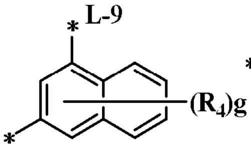
L-10
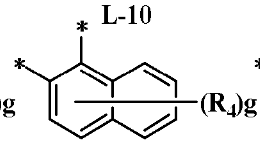
L-11
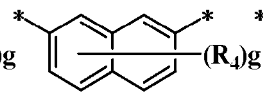
L-12
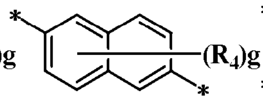
L-13
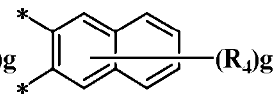

In Structural Formulas L-1 to L-13,

$R_4$ is each independently the same as the definition of $R_3$ described above,
f is each independently an integer from 0 to 4,
g may be each independently an integer from 0 to 6, and
* means a position bonded to $L_1$ or $L_2$.

According to one embodiment of the present disclosure, a may be an integer from 0 to 3, and more specifically, 0, 1, or 2.

According to one embodiment of the present disclosure, b and c may be each independently an integer from 0 to 2, and more specifically, 0 or 1. Accordingly, b+c may be an integer from 0 to 2.

According to one embodiment of the present disclosure, d may be specifically 0 or 1.

According to one embodiment of the present disclosure, e may be specifically 0 or 1.

According to one embodiment of the present disclosure, h may be specifically 0 or 1.

According to one embodiment of the present disclosure, f and g may be specifically 0 or 1.

According to one embodiment of the present disclosure, Chemical Formula 1 above may be represented by one of Chemical Formulas 2 to 14 below.

<Chemical Formula 2>

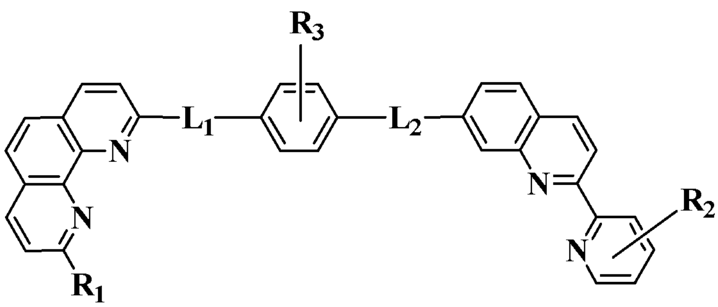

<Chemical Formula 3>

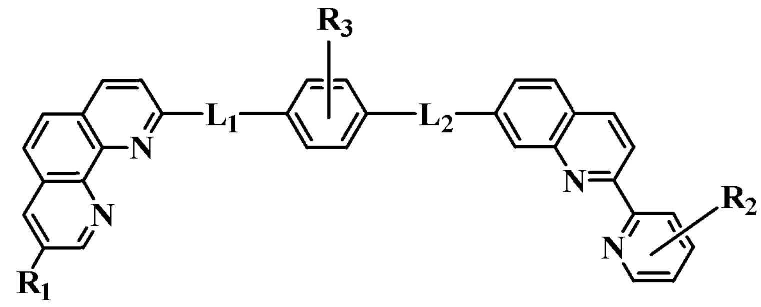

<Chemical Formula 4>

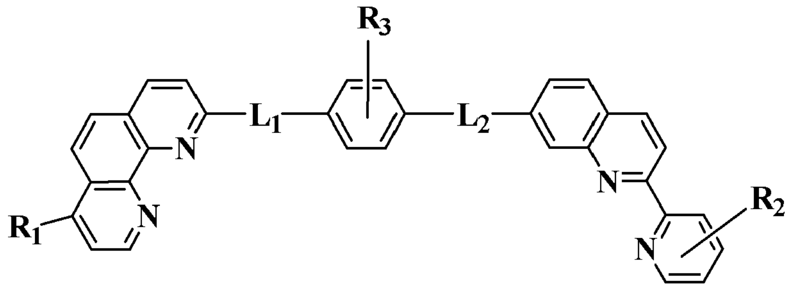

<Chemical Formula 5>

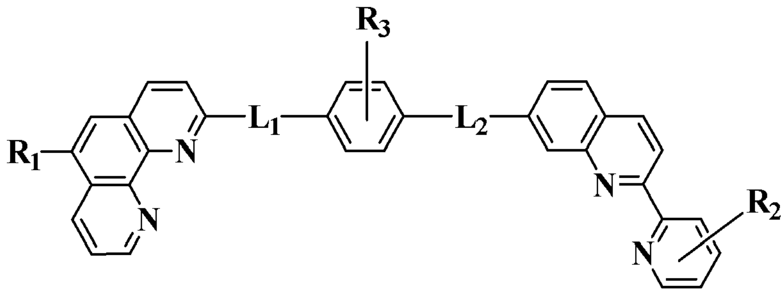

<Chemical Formula 6>

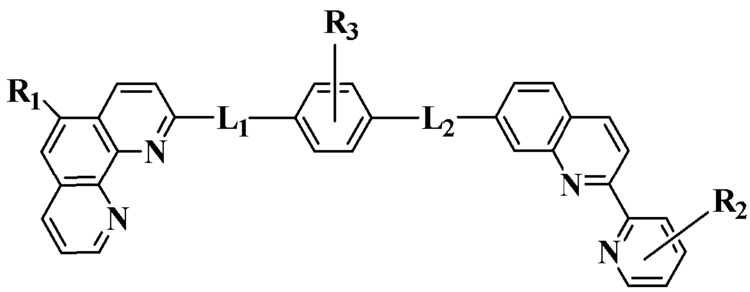

<Chemical Formula 7>

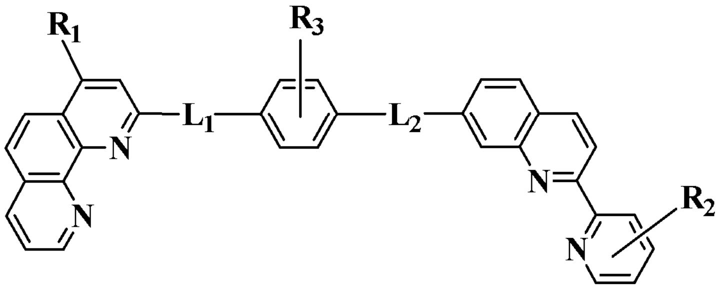

<Chemical Formula 8>

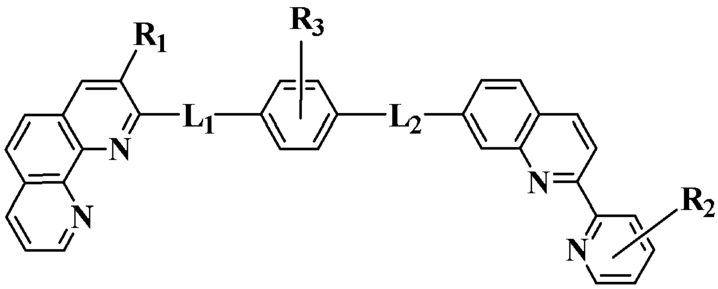

<Chemical Formula 9>

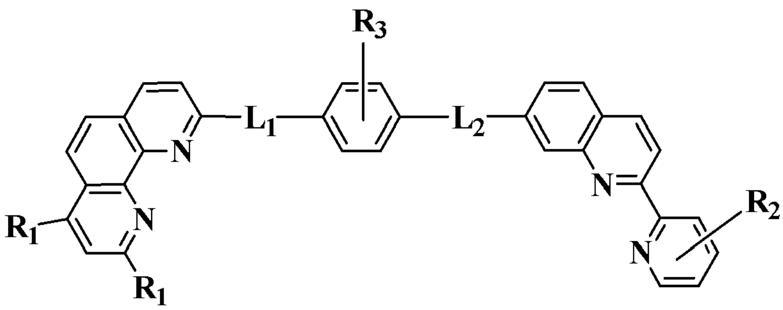

<Chemical Formula 10>

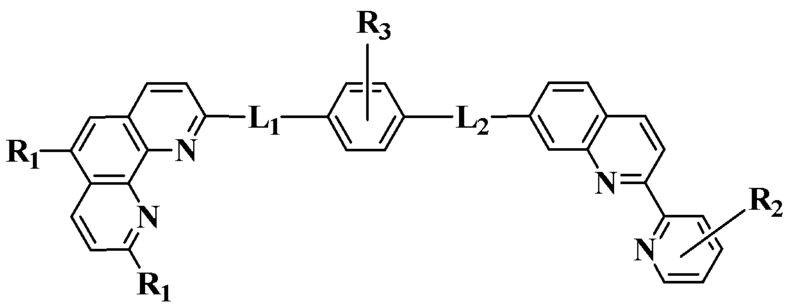

<Chemical Formula 11>

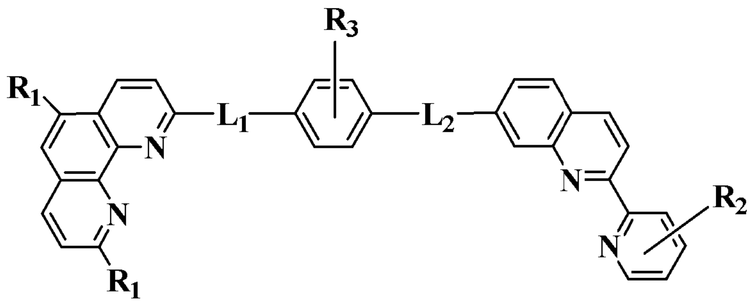

<Chemical Formula 12>

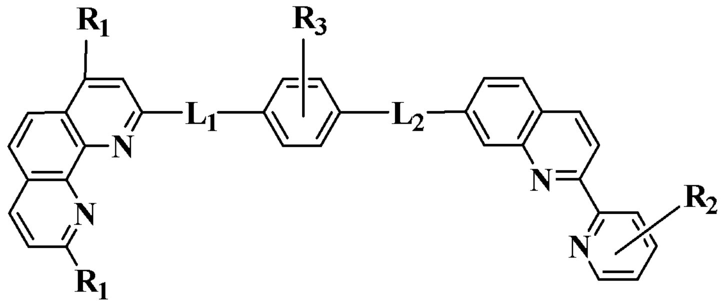

<Chemical Formula 13>

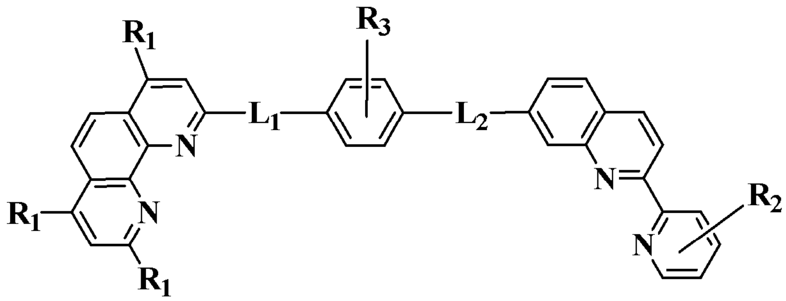

<Chemical Formula 14>

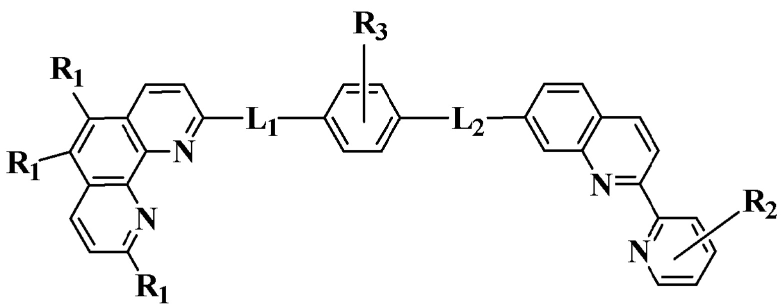

In Chemical Formulas 2 to 14,

$R_1$ is each independently one of hydrogen, a phenyl group, and a pyridine group,
$R_2$ is each independently one of hydrogen, a cyano group, a phenyl group, and a pyridine group,
$R_3$ is each independently one of hydrogen, a cyano group, and a phenyl group, and
$L_1$ and $L_2$ are each selected from the group consisting of a direct bond, a phenylene group, and a naphthylene group, and $L_1$ and $L_2$ are substituted or unsubstituted with one of a cyano group and a phenyl group.

According to one embodiment of the present disclosure, $L_1$ may be a direct bond. That is, the linker that is the most adjacent to 1,10-phenanthroline may be 1,4-phenylene.

According to one embodiment of the present disclosure, $R_1$ may be hydrogen or a phenyl group, and when $R_1$ is a phenyl group, it may be preferable in terms of thermal stability.

According to one embodiment of the present disclosure, the compound of the present disclosure has a low reorganization energy (RE) value $\lambda_{electron}$ of a certain level or less, and at the same time, can satisfy a certain level or higher of a movement rate constant ratio of holes and electrons $k_{et}(e)/k_{et}(h)$. In this way, a low RE value of a certain level or lower and a large $k_{et}(e)/k_{et}(h)$ of a certain level or higher are simultaneously satisfied, it can be effective in increasing the efficiency of the device as a result. According to one embodiment, the $k_{et}(e)/k_{et}(h)$ of the compound of the present disclosure may be 1.0 or more, more specifically, more than 1.0, and even more specifically, 1.10 or more.

In this specification, the RE value is calculated as (electron extraction potential, EEP) - (electron affinity, EA), where EEP is the energy when a structure in an anionic state becomes a cation, and EA refers to the anion energy in the ground state (neutral).

In addition, in this specification, the $k_{et}(e)$ value is the electron movement rate constant, the $k_{et}(h)$ value is the hole movement rate constant, and the movement rate constant ratio of holes and electrons $k_{et}(e)/k_{et}(h)$ may be calculated according to Equation 1 below.

[Equation 1]

$$\frac{k_{et}(e)}{k_{et}(h)} = \frac{\sqrt{4\pi * \lambda_{hole} * 298.15K} * exp\left(-\frac{\lambda_{electron}}{4 * 8.6173324 * 10^{-5} * 298.15K}\right)}{\sqrt{4\pi * \lambda_{electron} * 298.15K} * exp\left(-\frac{\lambda_{hole}}{4 * 8.6173324 * 10^{-5} * 298.15K}\right)}$$

In Equation 1, $8.6173324*10^{-5}$ (eV/(degree K)) is the Boltzmann constant.

Meanwhile, the compound of the present disclosure according to one embodiment of the present disclosure is characterized by satisfying high glass transition temperature (Tg) and decomposition temperature (Td) of a certain level or higher, thereby having thermal stability.

Specifically, the compound represented by Chemical Formula 1 of the present disclosure may be selected from the group consisting of Compounds 1 to 137 below. Such compounds are merely one examples, and the compound of the present disclosure is not limited thereto.

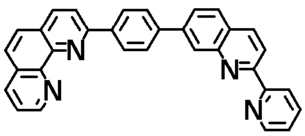

1

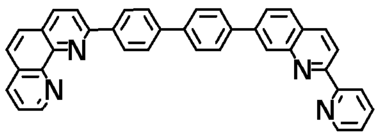

2

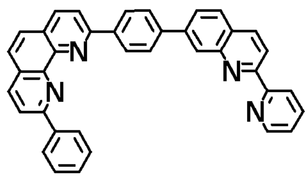

3

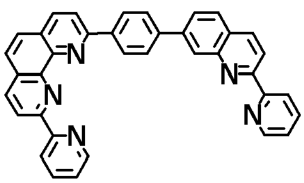

4

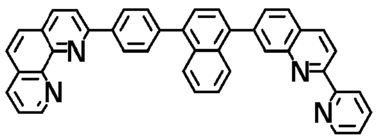

5

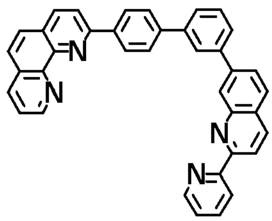

6

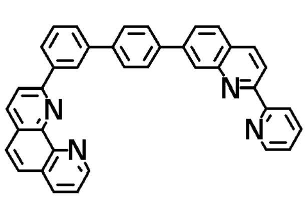

**7**

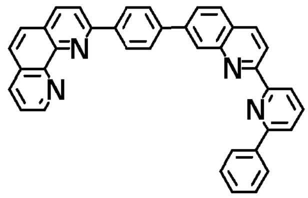

**8**

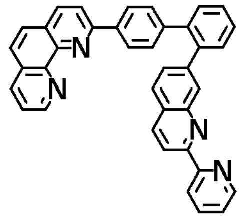

**9**

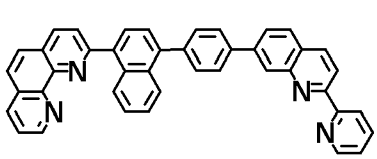

**10**

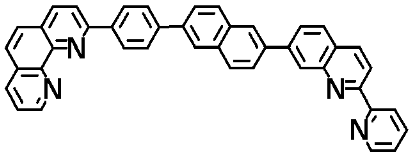

**11**

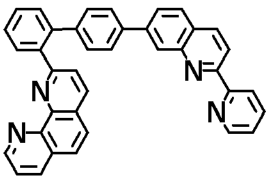

**12**

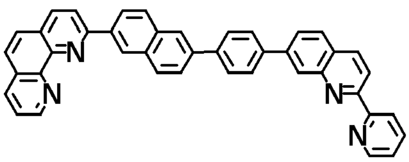

**13**

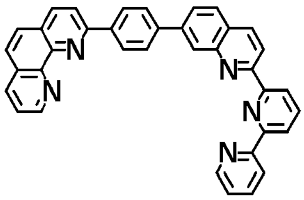

**14**

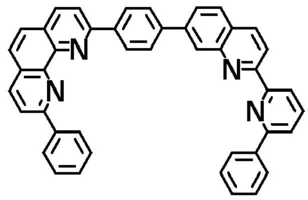

**15**

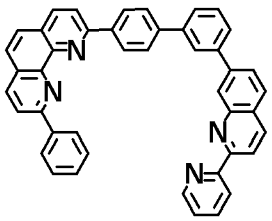

**16**

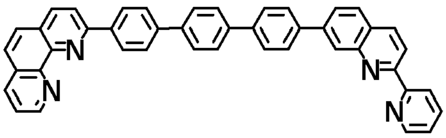

**17**

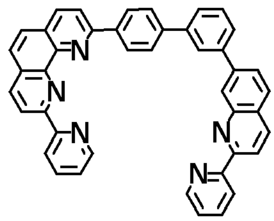

**18**

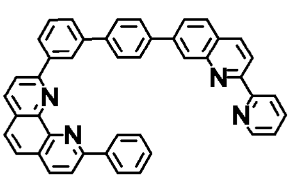

**19**

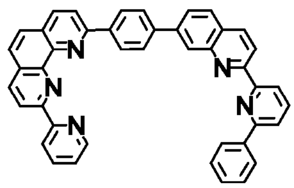

**20**

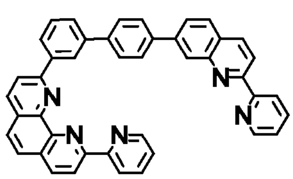

**21**

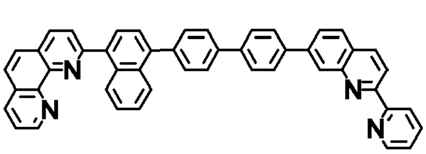

**22**

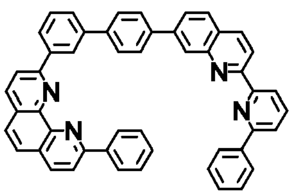

**23**

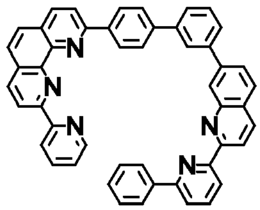

**24**

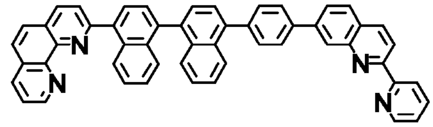

**25**

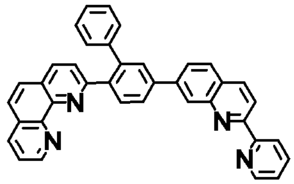

**26**

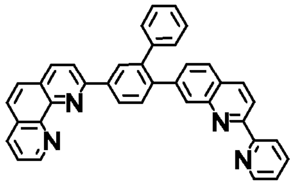

**27**

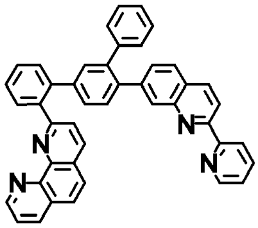

**28**

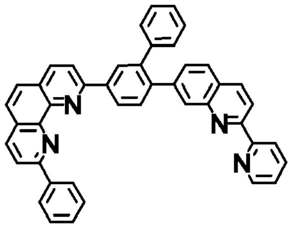

**29**

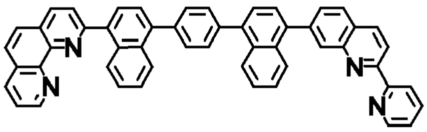

**30**

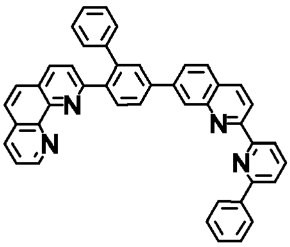

**31**

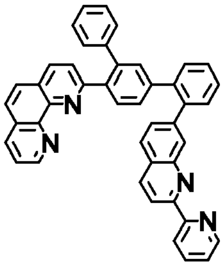

**32**

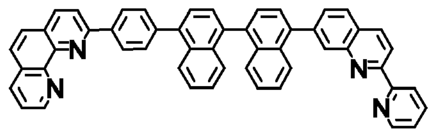

**33**

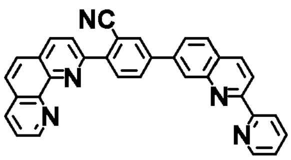

**34**

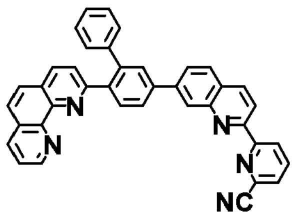

**35**

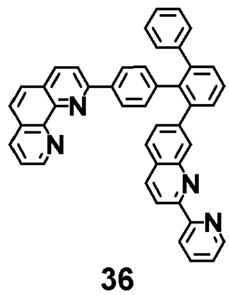

**36**

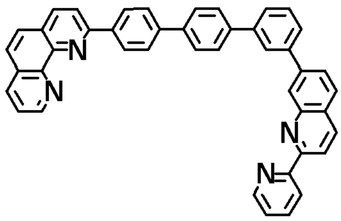

**37**

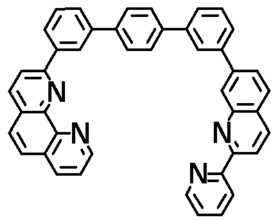

**38**

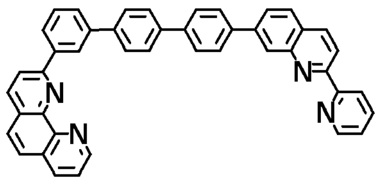

**39**

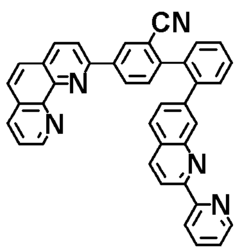

**40**

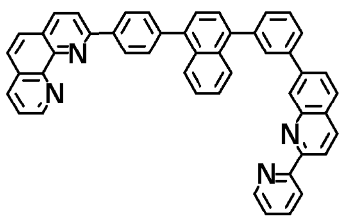

**41**

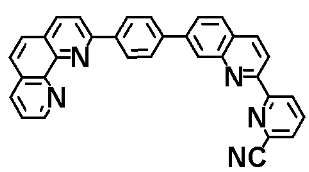

**42**

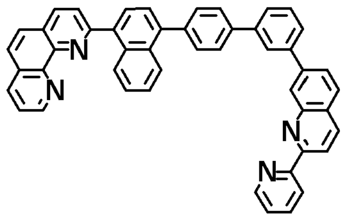

43

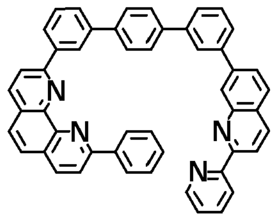

44

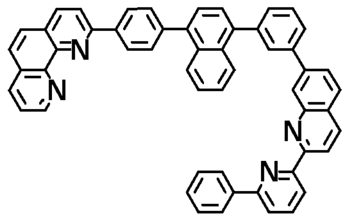

45

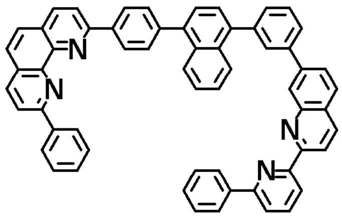

46

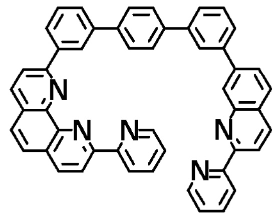

47

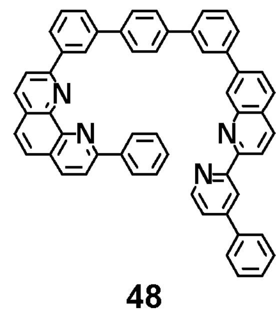

48

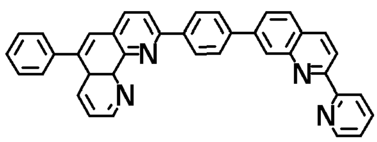

49

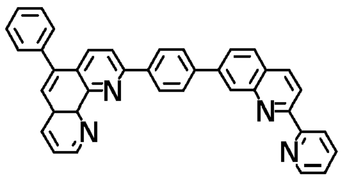

50

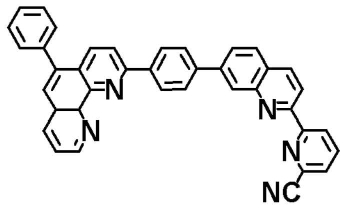

51

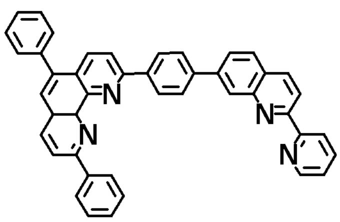

52

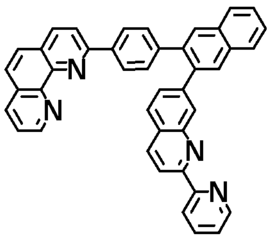

53

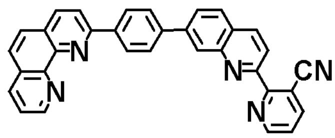

54

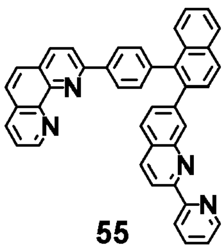

55

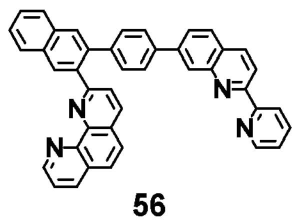

56

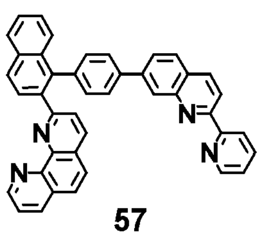

57

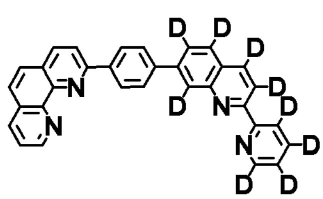

58

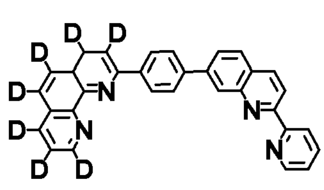

59

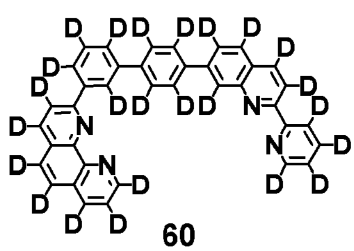

60

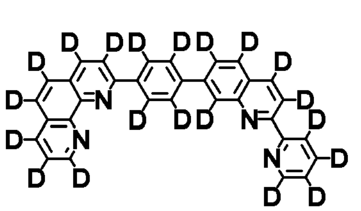
61

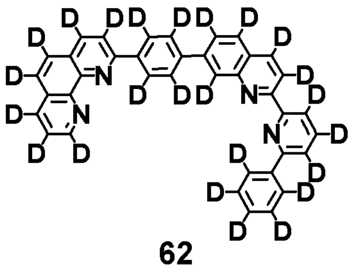
62

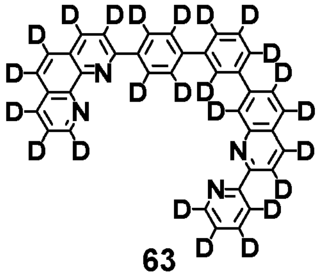
63

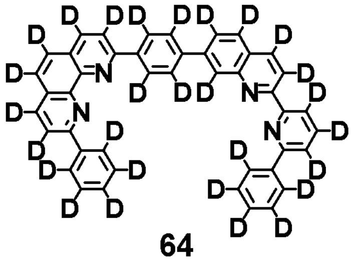
64

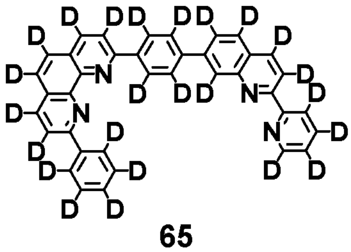
65

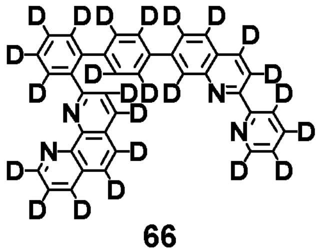
66

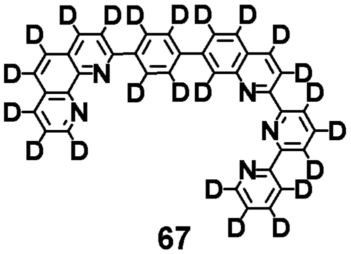
67

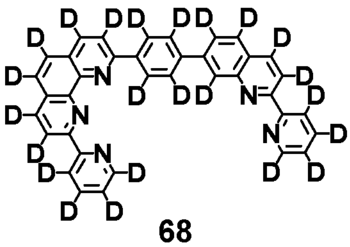
68

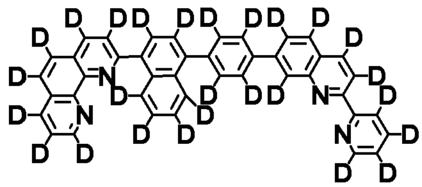
69

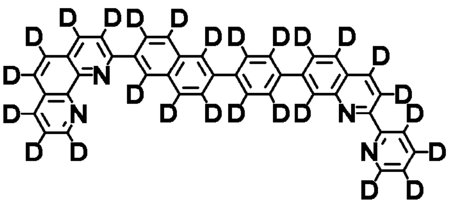
70

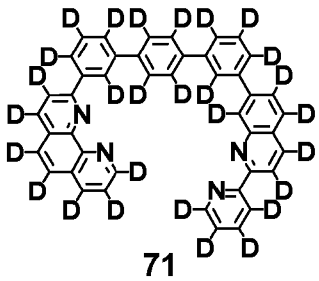
71

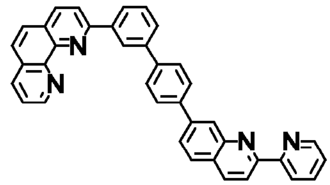
72

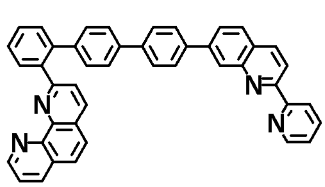
73

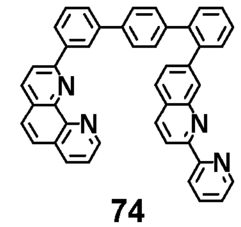
74

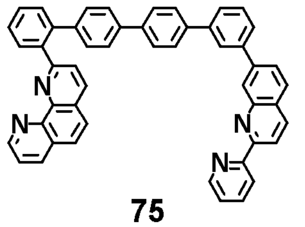
75

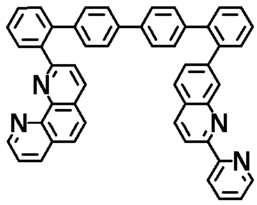
76

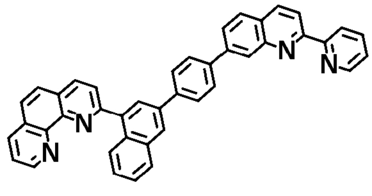
77

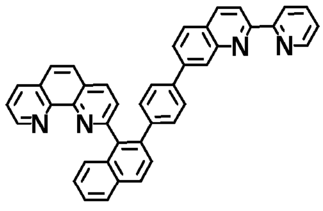
78

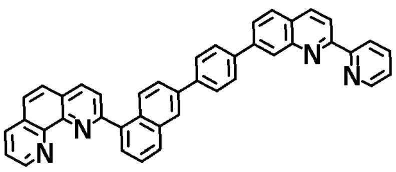

**79**

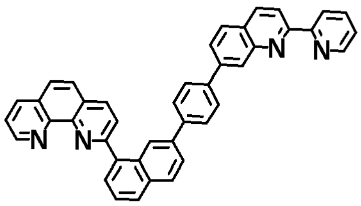

**80**

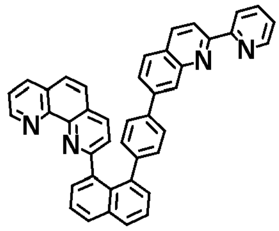

**81**

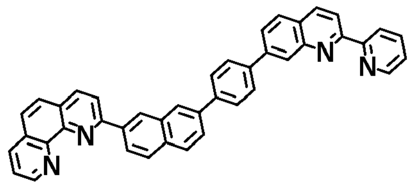

**82**

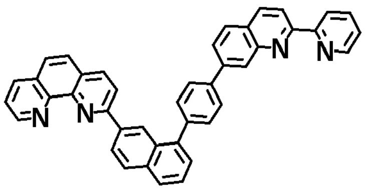

**83**

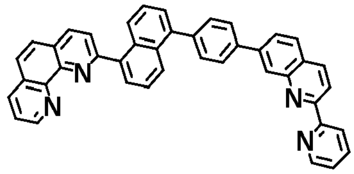

**84**

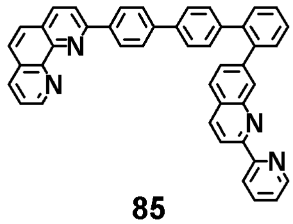

**85**

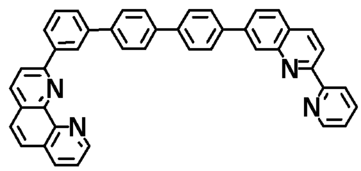

**86**

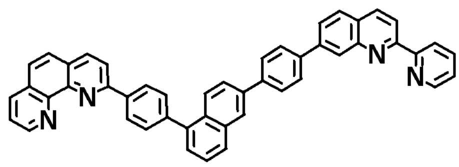

**87**

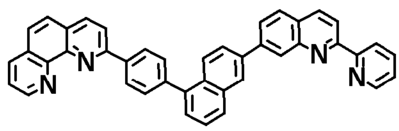

**88**

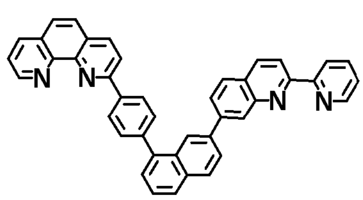

**89**

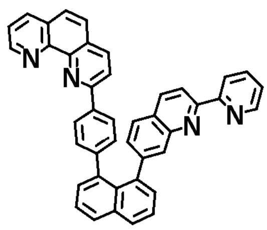

**90**

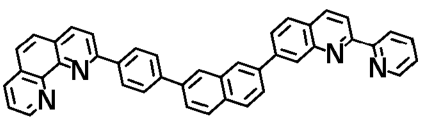

**91**

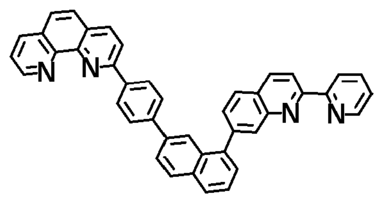

**92**

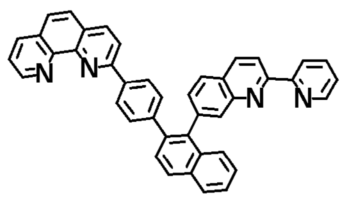

**93**

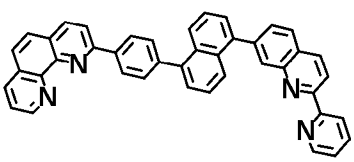

**94**

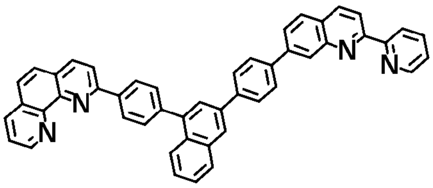

**95**

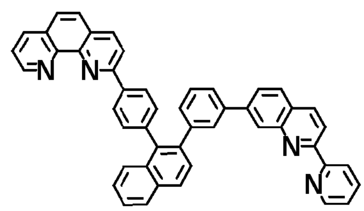

**96**

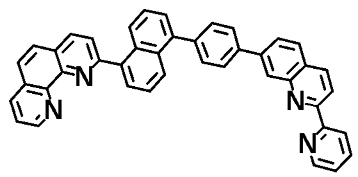

**97**

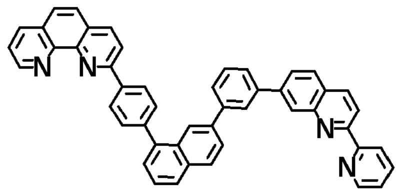

**98**

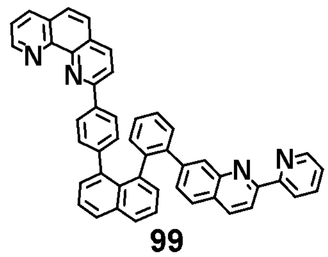

**99**

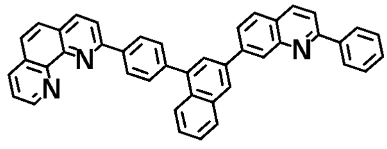

**100**

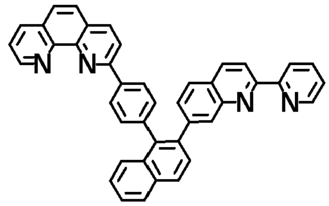

**101**

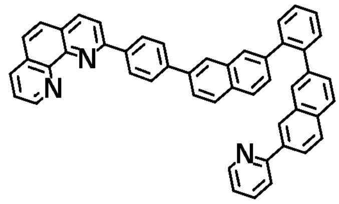

**102**

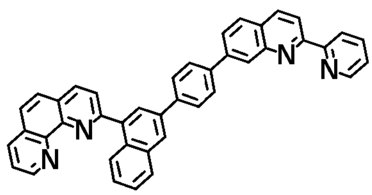

**103**

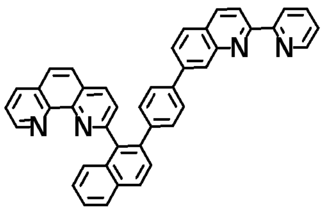

**104**

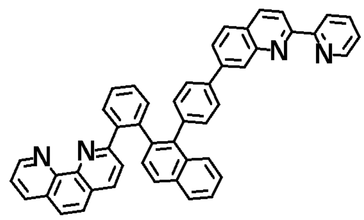

**105**

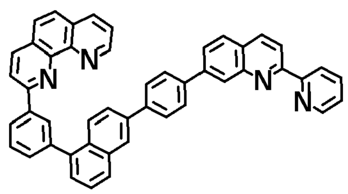

**106**

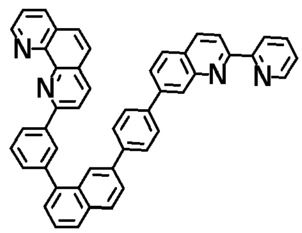

**107**

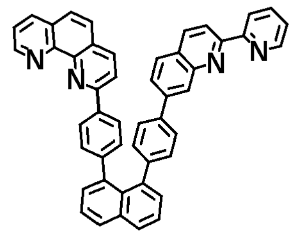

**108**

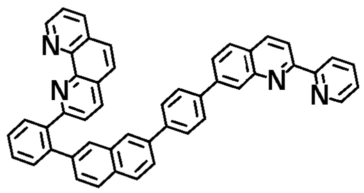

**109**

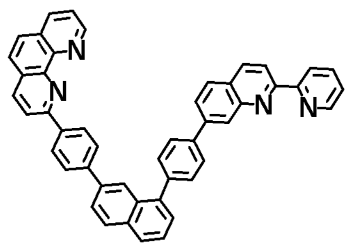

**110**

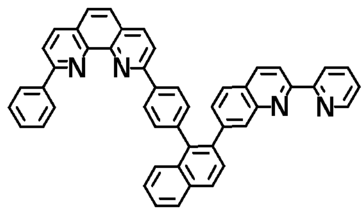

**111**

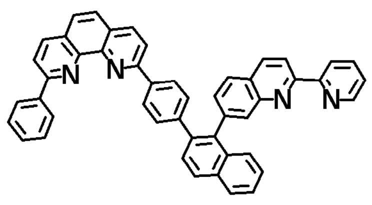

**112**

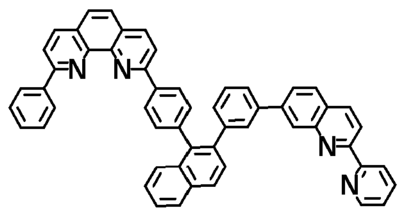

**113**

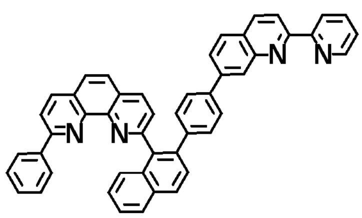

**114**

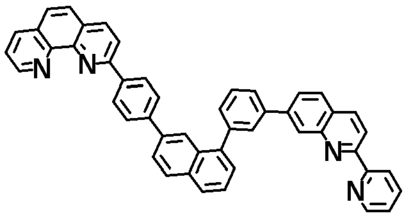
115

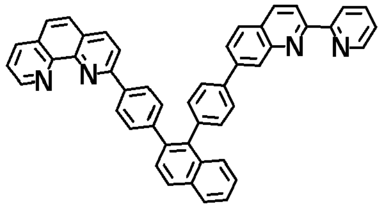
116

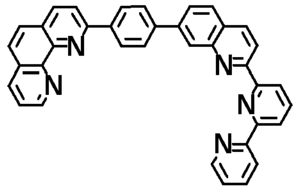
117

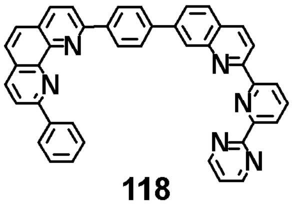
118

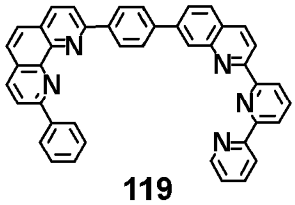
119

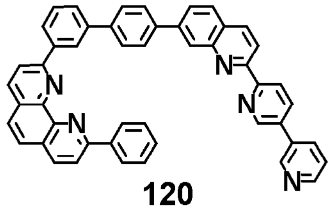
120

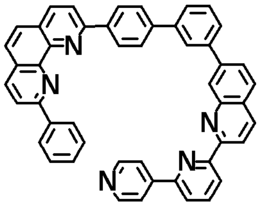
121

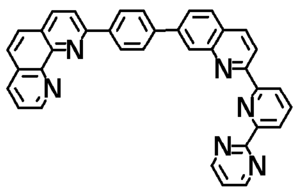
122

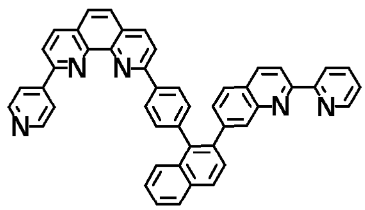
123

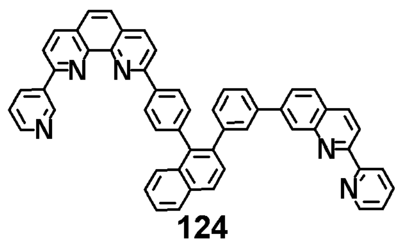
124

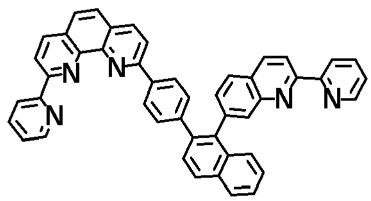
125

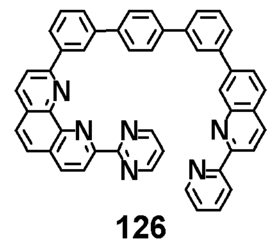
126

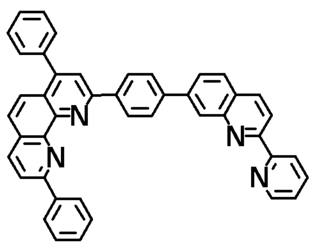
127

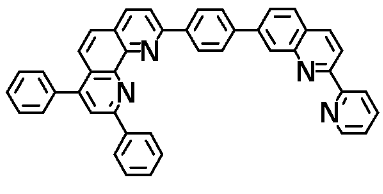
128

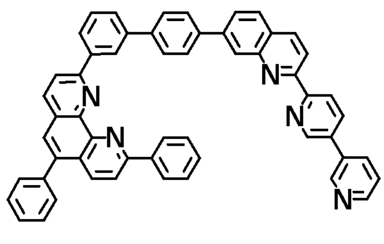
129

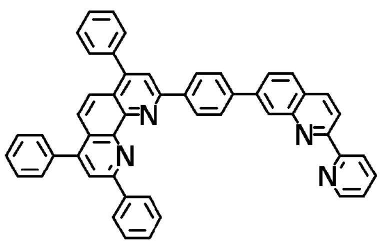
130

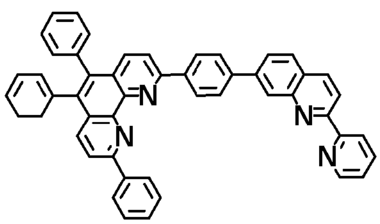
131

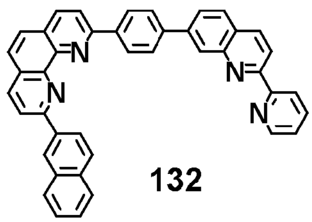
132

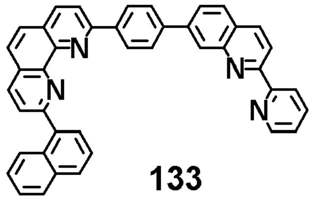
133

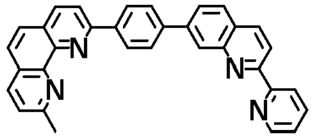
134

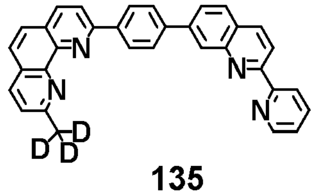
135

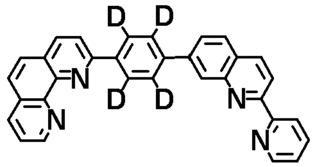
136

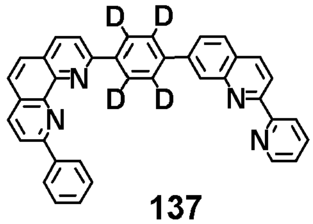
137

The compound of the present disclosure described so far is a compound in which 1,10-phenanthroline and quinoline-pyridine are bonded, and more specifically, it is characterized in that quinoline-pyridine is bonded at the position right next(adjacent) to N of 1,10-phenanthroline (structural feature 1), pyridine is bonded at the 2nd position right next to N of quinoline (structural feature 2), 1,10-phenanthroline is bonded at the 7th position of quinoline (structural feature 3), when pyridine is bonded to quinoline, it is bonded at the position right next to N of pyridine (structural feature 4), and one or more 1,4-phenylene are included as a linker between 1,10-phenanthroline and quinoline-pyridine (structural feature 5), and at the same time, the linker between 1,10-phenanthroline and quinoline-pyridine is all composed of an arylene group (structural feature 6).

It is preferable that such a new compound of the present disclosure is applied to an organic material layer to which an electron-transporting material is applied among the organic material layers within an organic light emitting device. In particular, when applied to a charge generation layer (CGL), particularly an n-type charge generation layer (nCGL), within a tandem-type organic light emitting device, it can exhibit effects such as increased device efficiency, reduced driving voltage, longer lifespan, etc. Meanwhile, the new compound of the present disclosure is not limited thereto, and can also be applied to organic material layers such as an electron injection layer (EIL), an electron transport layer (ETL), a hole blocking layer (HBL), and a layer that simultaneously injects and transports electrons to which compounds with excellent electron mobility have to be applied.

The present disclosure provides an organic light emitting device including a first electrode and a second electrode, and one or more organic material layers disposed between the first electrode and the second electrode, wherein one or more of the organic material layers contain the compound according to Chemical Formula 1 above.

Furthermore, the present disclosure provides a tandem-type organic light emitting device including a first electrode and a second electrode, a plurality of emission units positioned between the first electrode and the second electrode, and charge generation layers which are each positioned at one or more positions between two emission units that are adjacent to each other, wherein one or more of the charge generation layers include an N-type charge generation layer containing the compound represented by Chemical Formula 1 above.

Hereinafter, the organic light emitting device and the tandem-type organic light emitting device according to the present disclosure will be described in more detail.

The organic light emitting device has an organic material layer positioned between the first electrode and the second electrode. The organic material layer may be formed by including one or more organic material layers, and specifically, may be formed by including one or more layers selected from known organic material layers constituting an emission unit, such as a hole injection layer (HIL), a hole transport layer (HTL), an emission layer (EML), an electron transport layer (ETL), an electron injection layer (EIL), etc.

The hole injection layer (HIL) is a layer that injects holes from an electrode, and a compound which has the ability to transport holes, thereby having a hole injection effect at the anode and an excellent hole injection effect for the emission layer or emission material, which prevents the movement of excitons generated in the emission layer to the electron injection layer or electron injection material, and which also has excellent thin-film forming ability is preferable as a hole injection material.

The hole transport layer (HTL) is a layer that receives holes from the hole injection layer and transports the holes to the emission layer, and a material with high mobility for holes as a material which can receive the holes from the anode or the hole injection layer and transport them to the emission layer is suitable for the hole transport material.

The emission layer (EML) is a layer that emits light through the recombination of electrons and holes, and a material with high quantum efficiency for fluorescence or phosphorescence, as a material that can emit light in the visible light range by receiving holes and electrons from the hole transport layer and electron transport layer, respectively, and

combining them, is preferable as an emission material. The emission layer may specifically include a host and a dopant.

The electron transport layer (ETL) is a layer that receives electrons from the electron injection layer and transports the electrons to the emission layer, and a material with high mobility for electrons, as a material which can well receive electrons from the cathode and transport the electrons to the emission layer, is preferable as an electron transport material.

The electron injection layer (EIL) is a layer that injects electrons from the electrode, and a compound which has a capability of transporting electrons, has electron injection effects from the cathode and excellent electron injection effects for the emission layer or emission material, prevents excitons generated in the emission layer from moving to the hole injection layer, and further has excellent thin-film formation capability is preferable.

Meanwhile, the organic material layer may further include a hole blocking layer (HBL). The hole blocking layer may be positioned between the emission layer and the electron transport layer and reduce the problem of holes of the hole injection layer invading the electron transport layer. The hole blocking layer is also one of layers of the electron transport region, and a material with capability of transporting electrons is suitable.

Meanwhile, the organic material layer may include a layer that simultaneously injects and transports electrons.

The new compound according to Chemical Formula 1 of the present disclosure in the organic material layers of such an organic light emitting device is a material with excellent electron transport capability, and is preferably applied to one or more of the electron injection layer, electron transport layer, hole blocking layer, and layers that simultaneously inject and transport electrons, in the electron transport region.

Meanwhile, the configuration of the organic light emitting device may be variously changed or altered. According to one embodiment, the organic light emitting device may be a tandem-type organic light emitting device in which two or more plural emission parts (or emission units) including an emission layer are provided to be stacked between a first electrode and a second electrode, and in the case of such a tandem structure, in addition to the above-mentioned organic material layers, a charge generation layer (CGL) that adjusts charge balance may be further included as one of the organic material layers, and may be positioned at each of one or more locations between two emission parts that are adjacent to each other. Such a charge generation layer is composed of a plurality of layers including an N-type charge generation layer that plays a role of injecting electrons and a P-type charge generation layer that plays a role of injecting holes, but is not limited thereto and may be composed of a single layer.

The new compound of the present disclosure may also be applied to the charge generation layer of such a tandem-type organic light emitting device, and specifically, may be applied to the N-type charge generation layer. The N-type charge generation layer may be formed of an organic material layer doped with a dopant such as a metal or the like, and specifically, may be formed by containing a host and a dopant. At this time, the new compound of the present disclosure may be applied as a host, and the compound of the present disclosure can smoothly perform interaction with the dopant by including 1,10-phenanthroline and quinoline-pyridine having an unshared pair of electrons. Meanwhile, the dopant may be a material including a metal element, and specifically, may be one or more of a metal, a metal compound, and an organic complex of a metal. Here, the metal element may be one or more selected from lithium (Li), sodium (Na), potassium (K), rubidium (Rb), cesium (Cs), beryllium (Be), magnesium (Mg), calcium (Ca), strontium (Sr), barium (Ba), ytterbium (Yb), samarium (Sm), tin (Sn), copper (Cu), titanium (Ti), cadmium (Cd), mercury (Hg), lead (Pb), bismuth (Bi), zinc (Zn), iron (Fe), cobalt (Co), nickel (Ni), indium (In), gallium (Ga), thorium (Th), uranium (U), silver (Ag), aluminum (Al), gold (Au), molybdenum (Mo), niobium (Nb), palladium (Pd), platinum (Pt), and europium (Eu), and may be lithium or ytterbium according to one embodiment. Such dopants may be doped at a ratio of 0.1 to 20% by weight relative to the total host material, specifically 0.5 to 15% by weight, but are not limited thereto and may vary depending on the types of metal elements.

According to an embodiment of the present disclosure, when ytterbium, which has a work function lower than lithium, is included as a dopant in the N-type charge generation layer, it may be preferable to use a host in which a LUMO value satisfies a relatively deep level of -1.8 eV to -1.9 eV in terms of smooth electron movement and low driving implementation. According to one embodiment, when $L_1$ and $L_2$ in Chemical Formula 1 are direct bonds, and $R_1$ to $R_3$ and $R_5$ do not contain nitrogen atoms, the LUMO value at the above level may be satisfied, and specifically, a compound in which $R_1$ to $R_3$ are each hydrogen or a phenyl group, and $R_5$ is hydrogen may be suitable.

Such a compound of the present disclosure may be applied to the electron transport layer, hole blocking layer, or charge generation layer of the organic light emitting device, and particularly, the compound can readily bind to dopants such as Li and Yb when the compound is applied to the N-type charge generation layer by including 1,10-phenanthroline with an unshared pair of electrons. Further, the unshared pair of electrons on nitrogens in quinoline-pyridine more easily help bonding with dopants, thereby making it desirable for them to be used also as a host for N-type charge generation layers using dopants such as not only Li but also Yb. Further, the compound of the present disclosure has the effect of increasing device efficiency as a result by having a low reorganization energy (RE) value of a certain level or lower and simultaneously satisfying the rate constant ratio of holes and electrons ($k_{et}(e)/k_{et}(h)$) of a certain level or higher at the same time. At the same time, the compound of the present disclosure has characteristics in which thermal stability is excellent through a structure which includes 1,4-phenylene as a linker.

Hereinafter, the present disclosure will be described in more detail in the following sections: Synthetic Examples, Experimental Examples, and Examples. However, the following contents do not limit the scope of the present disclosure.

## Synthesis of Compounds

### Synthetic Example 1. Synthesis of Compound 1

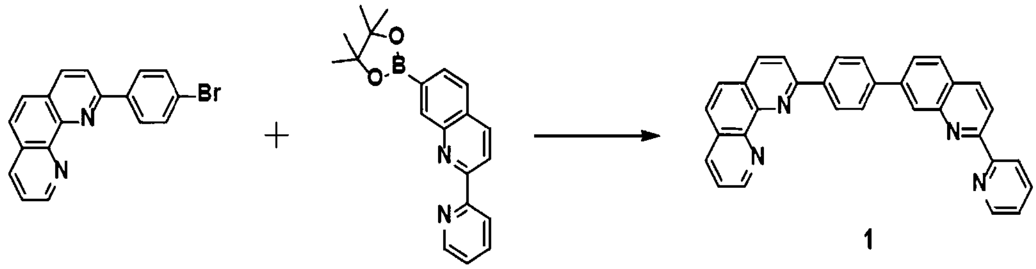

15.0 g (44.75 mmol) of 2-(4-bromophenyl)-1,10-phenanthroline, 16.4 g (9.22 mmol) of 2-(pyridin-2-yl)-7-(4,4,5,5-tetramethyl-1,3,2-dioxaborolan-2-yl)quinoline, 18.6 g (134.25 mmol) of potassium carbonate, and 2.6 g (2.24 mmol) of tetrakis(triphenylphosphine)palladium were added to a round-bottomed flask and stirred overnight while performing reflux in 150 mL of 1,4-dioxane and 75 mL of water. Upon completion of the reaction, the mixture was cooled to room temperature, and then EtOH was added so that the solid was filtered and washed with EtOH and water. The solid was dissolved in dichlorobenzene, and then filtered under reduced pressure through silica gel and Celite to obtain a filtrate. The obtained filtrate was concentrated under reduced pressure and then recrystallized from dichlorobenzene to obtain 15.2 g (73.8%) of Compound 1.

### Synthetic Example 2. Synthesis of Compound 3

Synthesis of Intermediate 1

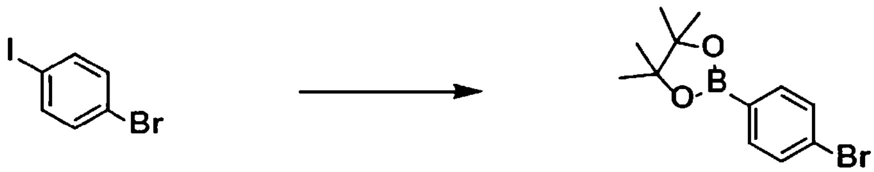

20.0 g (70.69 mmol) of 1-bromo-4-iodobenzene, 21.5 g (84.83 mmol) of bis(pinacolato)diboron, 20.8 g (212.08 mmol) of potassium acetate, and 1.7 g (2.12 mmol) of [1,1'-Bis(diphenylphosphino)ferrocene]dichloropalladium(II) were added to a round-bottomed flask and stirred overnight while performing reflux in 300 mL of 1,4-dioxane. Upon completion of the reaction, the mixture was cooled to room temperature, and then concentrated under reduced pressure to obtain a filtrate. The obtained filtrate was extracted using ethyl acetate and water. The organic material layer was dehydrated with $MgSO_4$ and concentrated under reduced pressure to obtain a filtrate. The obtained filtrate was dissolved in dichloromethane, filtered through silica gel and Celite under reduced pressure, and concentrated under reduced pressure to obtain 17.6 g (88.0%) of Intermediate 1.

Synthesis of Intermediate 2

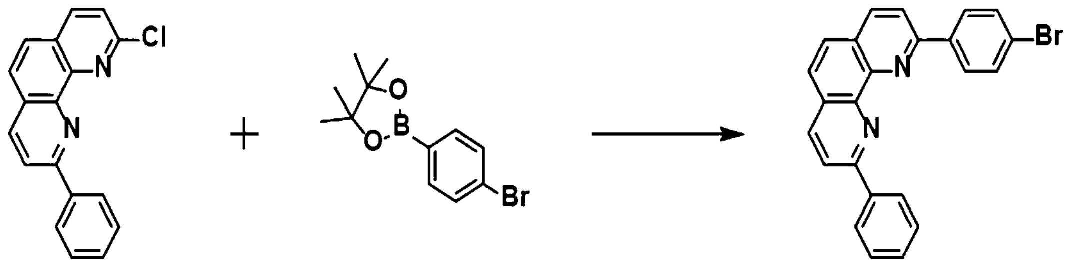

15.0 g (51.59 mmol) of 2-chloro-9-phenyl-1,10-phenanthroline, 16.1 g (56.75 mmol) of Intermediate 1, 21.4 g (154.77 mmol) of potassium carbonate, and 3.0 g (2.58 mmol) of tetrakis(triphenylphosphine)palladium were added to a round-bottomed flask and stirred overnight while performing reflux in 150 mL of 1,4-dioxane and 75 mL of water. Upon completion of the reaction, the mixture was cooled to room temperature, and then EtOH was added so that the solid was filtered and washed with EtOH and water. The solid was dissolved in toluene, and then filtered under reduced pressure through silica gel and Celite to obtain a filtrate. The obtained filtrate was concentrated under reduced pressure and then recrystallized from toluene to obtain 15.2 g (73.8%) of Intermediate 2.

Synthesis of Compound 3

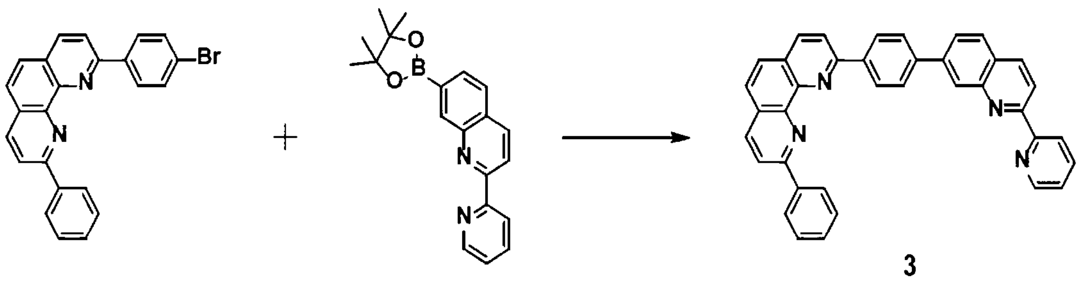

3

14.5 g (35.25 mmol) of Intermediate 2 and 12.9 g (38.78 mmol) of 2-(pyridin-2-yl)-7-(4,4,5,5-tetramethyl-1,3,2-dioxaborolan-2-yl)quinoline, 14.6 g (105.76 mmol) of potassium carbonate, and 2.0 g (1.76 mmol) of tetrakis(triphenylphosphine)palladium were added to a round-bottomed flask and stirred overnight while performing reflux in 140 mL of 1,4-dioxane and 70 mL of water. Upon completion of the reaction, the mixture was cooled to room temperature, and then EtOH was added so that the solid was filtered and washed with EtOH and water. The solid was dissolved in dichlorobenzene, and then filtered under reduced pressure through silica gel and Celite to obtain a filtrate. The obtained filtrate was concentrated under reduced pressure and then recrystallized from dichlorobenzene to obtain 13.1 g (69.2%) of Compound 3.

**Synthetic Example 3. Synthesis of Compound 6**

Synthesis of Intermediate 3

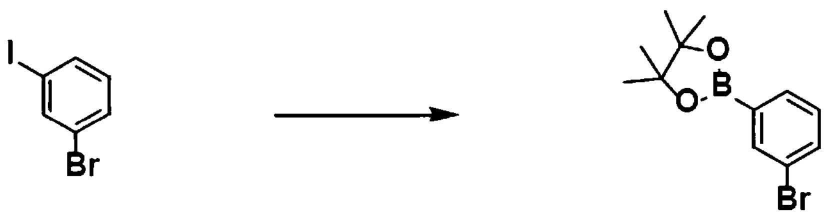

20.0 g (70.69 mmol) of 1-bromo-3-iodobenzene, 21.5 g (84.83 mmol) of bis(pinacolato)diboron, 20.8 g (212.08 mmol) of potassium acetate, and 1.7 g (2.12 mmol) of [1,1'-Bis(diphenylphosphino)ferrocene]dichloropalladium(II) were added to a round-bottomed flask and stirred overnight while performing reflux in 300 mL of 1,4-dioxane. Upon completion of the reaction, the mixture was cooled to room temperature, and then concentrated under reduced pressure to obtain a

filtrate. The obtained filtrate was extracted using ethyl acetate and water. The organic material layer was dehydrated with $MgSO_4$ and concentrated under reduced pressure to obtain a filtrate. The obtained filtrate was dissolved in dichloromethane, filtered through silica gel and Celite under reduced pressure, and concentrated under reduced pressure to obtain 15.9 g (79.5%) of Intermediate 3.

Synthesis of Intermediate 4

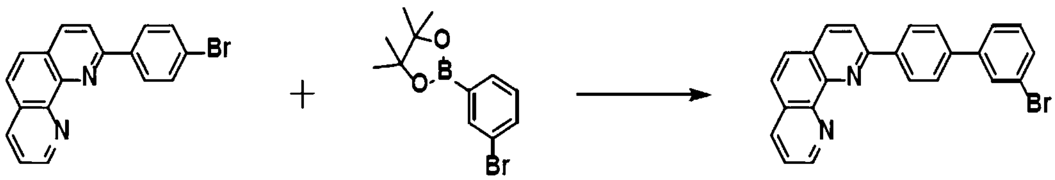

15.0 g (44.75 mmol) of 2-(4-bromophenyl)-1,10-phenanthroline, 13.9 g (49.22 mmol) of Intermediate 3, 18.6 g (134.25 mmol) of potassium carbonate, and 2.6 g (2.24 mmol) of tetrakis(triphenylphosphine)palladium were added to a round-bottomed flask and stirred overnight while performing reflux in 150 mL of 1,4-dioxane and 75 mL of water. Upon completion of the reaction, the mixture was cooled to room temperature, and then EtOH was added so that the solid was filtered and washed with EtOH and water. The solid was dissolved in toluene, and then filtered under reduced pressure through silica gel and Celite to obtain a filtrate. The obtained filtrate was concentrated under reduced pressure and then recrystallized from toluene to obtain 12.9 g (70.1%) of Intermediate 4.

Synthesis of Compound 6

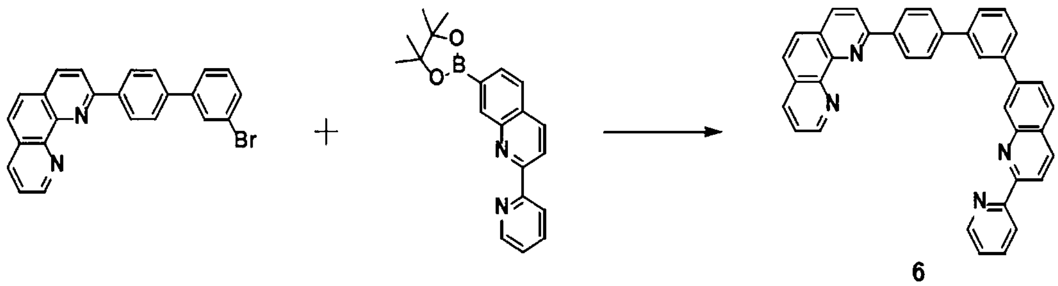

6

12.5 g (30.39 mmol) of Intermediate 4 and 11.1 g (33.43 mmol) of 2-(pyridin-2-yl)-7-(4,4,5,5-tetramethyl-1,3,2-dioxaborolan-2-yl)quinoline, 12.6 g (91.17 mmol) of potassium carbonate, and 1.8 g (1.52 mmol) of tetrakis(triphenylphosphine)palladium were added to a round-bottomed flask and stirred overnight while performing reflux in 120 mL of 1,4-dioxane and 60 mL of water. Upon completion of the reaction, the mixture was cooled to room temperature, and then EtOH was added so that the solid was filtered and washed with EtOH and water. The solid was dissolved in dichlorobenzene, and then filtered under reduced pressure through silica gel and Celite to obtain a filtrate. The obtained filtrate was concentrated under reduced pressure and then recrystallized from dichlorobenzene to obtain 10.9 g (66.8%) of Compound 6.

**Synthetic Example 4. Synthesis of Compound 16**

Synthesis of Intermediate 5

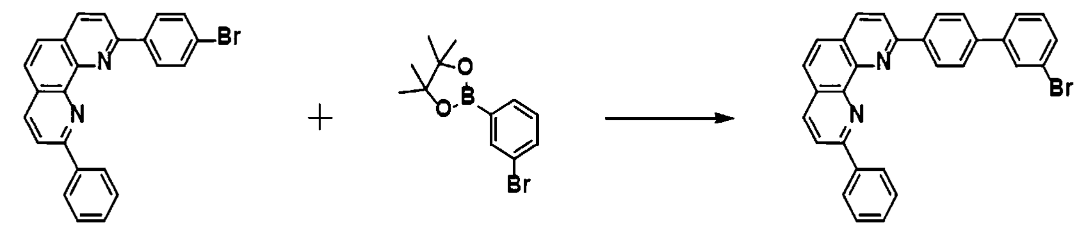

15.0 g (36.47 mmol) of Intermediate 2, 11.4 g (40.12 mmol) of 2-(3-bromophenyl)-4,4,5,5-tetramethyl-1,3,2-dioxaborolane, 15.1 g (109.41 mmol) of potassium carbonate, and 2.1 g (1.82 mmol) of tetrakis(triphenylphosphine) palladium were added to a round-bottomed flask and stirred overnight while performing reflux in 150 mL of 1,4-dioxane and 75 mL of water. Upon completion of the reaction, the mixture was cooled to room temperature, and then EtOH was added so that the solid was filtered and washed with EtOH and water. The solid was dissolved in toluene, and then filtered under reduced pressure through silica gel and Celite to obtain a filtrate. The obtained filtrate was concentrated under reduced pressure and then recrystallized from toluene to obtain 13.7 g (77.1%) of Intermediate 5.

Synthesis of Compound 16

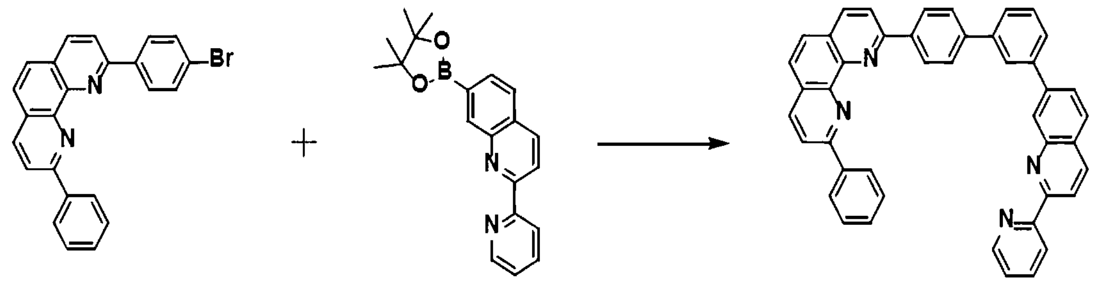

16

13.5 g (27.70 mmol) of Intermediate 5 and 10.1 g (30.47 mmol) of 2-(pyridin-2-yl)-7-(4,4,5,5-tetramethyl-1,3,2-dioxaborolan-2-yl)quinoline, 11.5 g (83.09 mmol) of potassium carbonate, and 1.6 g (1.38 mmol) of tetrakis(triphenylphosphine)palladium were added to a round-bottomed flask and stirred overnight while performing reflux in 130 mL of 1,4-dioxane and 65 mL of water. Upon completion of the reaction, the mixture was cooled to room temperature, and then EtOH was added so that the solid was filtered and washed with EtOH and water. The solid was dissolved in dichlorobenzene, and then filtered under reduced pressure through silica gel and Celite to obtain a filtrate. The obtained filtrate was concentrated under reduced pressure and then recrystallized from dichlorobenzene to obtain 11.4 g (67.2%) of Compound 16.

**Synthetic Example 5. Synthesis of Compound 20**

Synthesis of Intermediate 6

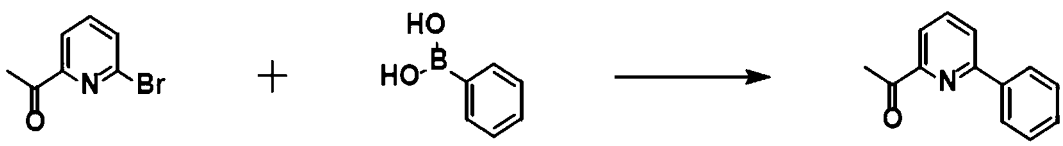

25.0 g (124.98 mmol) of 1-(6-bromopyridin-2-yl)ethan-1-one, 16.8 g (137.47 mmol) of phenylboronic acid, 51.8 g (374.93 mmol) of potassium carbonate, and 7.2 g (6.25 mmol) of tetrakis(triphenylphosphine)palladium were added to a round-bottomed flask and stirred overnight while performing reflux in 250 mL of 1,4-dioxane and 125 mL of water. Upon completion of the reaction, the mixture was cooled to room temperature, and then concentrated under reduced pressure to obtain a filtrate. The obtained filtrate was extracted using ethyl acetate and water. The organic material layer was dehydrated with $MgSO_4$ and then concentrated under reduced pressure to obtain a filtrate. The obtained filtrate was purified by column chromatography to obtain 19.6 g (79.5%) of Intermediate 6.

Synthesis of Intermediate 7

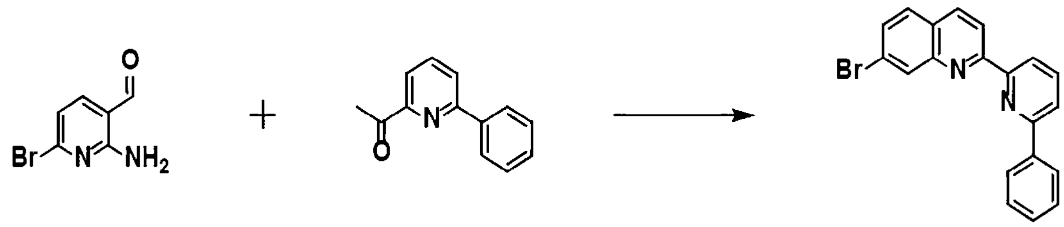

19.5 g (98.86 mmol) of Intermediate 6, 25.8 g (128.52 mmol) of 2-amino-6-bromonicotinaldehyde, and 135 mL oftoluene were added to a round-bottomed flask. After dissolving 16.6 g (296.59 mmol) of potassium hydroxide was dissolved in 95 mL of ethanol, potassium hydroxide dissolved in ethanol was added dropwise, and the mixture was stirred at 80°C overnight. Upon completion of the reaction, after the mixture was cooled to room temperature, methanol was added thereto to filter the mixture, and the solid was washed with methanol. The solid was dissolved in toluene, and then filtered under reduced pressure through silica gel and Celite to obtain a filtrate. The obtained filtrate was concentrated under reduced pressure and then recrystallized from toluene to obtain 19.5 g (54.6%) of Intermediate 7.

Synthesis of Intermediate 8

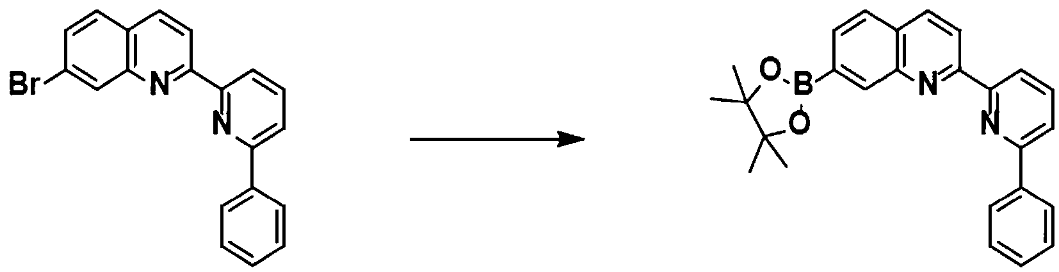

19.5 g (53.98 mmol) of Intermediate 7, 16.4 g (64.78 mmol) of bis(pinacolato)diboron, 15.9 g (161.94 mmol) of potassium acetate, and 1.3 g (1.62 mmol) of [1,1'-Bis(diphenylphosphino)ferrocene]dichloropalladium(II) were added to a round-bottomed flask and stirred overnight while performing reflux in 290 mL of 1,4-dioxane. Upon completion of the reaction, the mixture was cooled to room temperature and then concentrated under reduced pressure to obtain a filtrate. The obtained filtrate was extracted with ethyl acetate and water. The organic material layer was dehydrated with $MgSO_4$ and then concentrated under reduced pressure to obtain a filtrate. The obtained filtrate was dissolved in dichloromethane, filtered through silica gel and Celite under reduced pressure, and concentrated under reduced pressure to obtain 18.1 g (82.1%) of Intermediate 8.

Synthesis of Intermediate 9

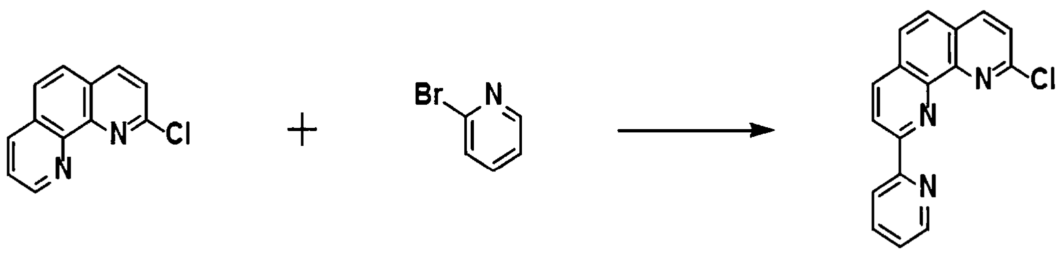

15.0 g (94.94 mmol) of 2-bromopyridine and 150 mL of THF were added to a round-bottomed flask and cooled to

-78°C, and then 62.3 mL (99.68 mmol) of n-BuLi in heptane was slowly added dropwise over 1 hour while performing stirring. After reaction for 1 hour, the temperature was raised to room temperature and stirring was performed for 2 hours. Thereafter, after the mixture was cooled to -78°C, 22.4 g (104.43 mmol) of 2-chloro-1,10-phenanthroline was slowly added thereto to perform reaction for 2 hours, and then the temperature was slowly raised to room temperature to perform stirring for 4 hours. Upon completion of the reaction, water was poured to quench the mixture, and then the water and organic material layers were separated to concentrate the organic material layer under reduced pressure. The obtained concentrate was extracted with dichloromethane and water. The organic material layer was dehydrated with $MgSO_4$ and concentrated under reduced pressure to obtain a filtrate. After the obtained filtrate was dissolved in 300 mL of dichloromethane, 35.5 g (408.23 mmol) of $MnO_2$ was added to the dissolved solution, and the mixture was stirred overnight while performing reflux. Upon completion of the reaction, the mixture was cooled to room temperature and the solid was filtered to remove $MnO_2$. Thereafter, water was added to the filtrate, and the mixture was extracted with dichloromethane and water. The organic material layer was dehydrated with $MgSO_4$ and then concentrated under reduced pressure. The obtained filtrate was recrystallized with EtOH to obtain 17.1 g (61.7%) of Intermediate 9.

Synthesis of Intermediate 10

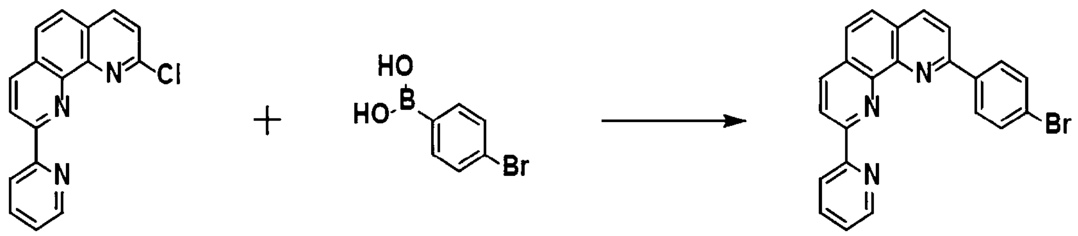

17.0 g (58.27 mmol) of Intermediate 9, 12.9 g (64.10 mmol) of (4-bromophenyl)boronic acid, 24.2 g (174.81 mmol) of potassium carbonate, and 3.4 g (2.91 mmol) of tetrakis(triphenylphosphine)palladium were added to a round-bottomed flask and stirred overnight while performing reflux in 170 mL of 1,4-dioxane and 85 mL of water. Upon completion of the reaction, the mixture was cooled to room temperature, and then EtOH was added so that the solid was filtered and washed with EtOH and water. The solid was dissolved in toluene, and then filtered under reduced pressure through silica gel and Celite to obtain a filtrate. The obtained filtrate was concentrated under reduced pressure and then recrystallized from toluene to obtain 16.4 g (68.3%) of Intermediate 10.

Synthesis of Compound 20

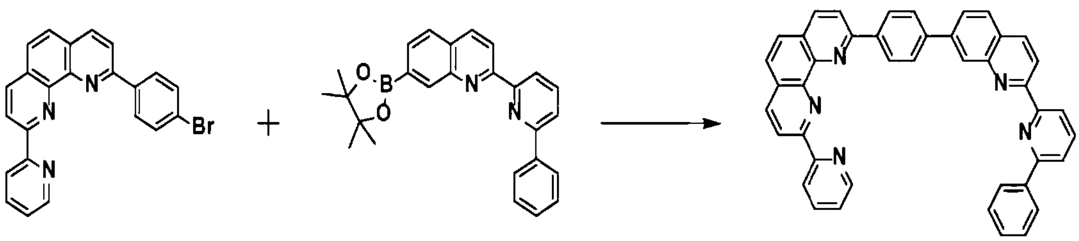

20

16.0 g (38.81 mmol) of Intermediate 10, 17.4 g (42.69 mmol) of Intermediate 8, 16.1 g (116.42 mmol) of potassium carbonate, and 2.2 g (1.94 mmol) of tetrakis(triphenylphosphine)palladium were added to a round-bottomed flask and stirred overnight while performing reflux in 160 mL of 1,4-dioxane and 80 mL of water. Upon completion of the reaction, the mixture was cooled to room temperature, and then EtOH was added so that the solid was filtered and washed with EtOH and water. The solid was dissolved in dichlorobenzene, and then filtered under reduced pressure through silica gel and Celite to obtain a filtrate. The obtained filtrate was concentrated under reduced pressure and then recrystallized from dichlorobenzene to obtain 14.2 g (59.6%) of Compound 20.

**Synthetic Example 6. Synthesis of Compound 29**

Synthesis of Intermediate 11

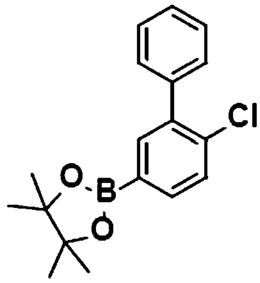

15.0 g (56.06 mmol) of 5-bromo-2-chloro-1,1'-biphenyl, 17.1 g (67.28 mmol) of bis(pinacolato)diboron, 16.5 g (168.19 mmol) of potassium acetate, and 1.4 g (1.68 mmol) of [1,1'-Bis(diphenylphosphino)ferrocene]dichloropalladium(II) were added to a round-bottomed flask and stirred overnight while performing reflux in 225 mL of 1,4-dioxane. Upon completion of the reaction, the mixture was cooled to room temperature, and then concentrated under reduced pressure to obtain a filtrate. The obtained filtrate was extracted using ethyl acetate and water. The organic material layer was dehydrated with $MgSO_4$ and then concentrated under reduced pressure to obtain a filtrate. The obtained filtrate was dissolved in dichloromethane, filtered through silica gel and Celite under reduced pressure, and concentrated under reduced pressure to obtain 15.7 g (89.0%) of Intermediate 11.

Synthesis of Intermediate 12

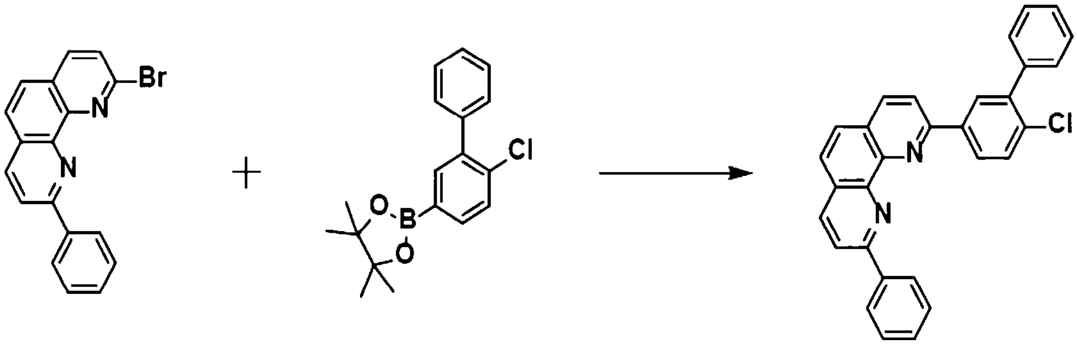

15.5 g (46.24 mmol) of 2-bromo-9-phenyl-1,10-phenanthroline, 16.0 g (50.87 mmol) of Intermediate 13, 19.2 g (138.72 mmol) of potassium carbonate, and 2.7 g (2.31 mmol) of tetrakis(triphenylphosphine)palladium were added to a round-bottomed flask and stirred overnight while performing reflux in 150 mL of 1,4-dioxane and 75 mL of water. Upon completion of the reaction, the mixture was cooled to room temperature, and then EtOH was added so that the solid was filtered and washed with EtOH and water. The solid was dissolved in toluene, and then filtered under reduced pressure through silica gel and Celite to obtain a filtrate. The obtained filtrate was concentrated under reduced pressure and then recrystallized from toluene to obtain 14.2 g (69.3%) of Intermediate 12.

Synthesis of Compound 29

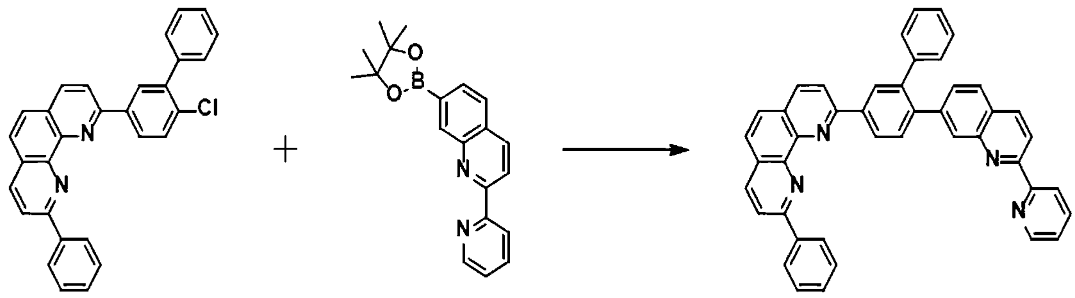

29

14.0 g (31.61 mmol) of Intermediate 12 and 11.5 g (34.77 mmol) of 2-(pyridin-2-yl)-7-(4,4,5,5-tetramethyl-1,3,2-dioxaborolan-2-yl)quinoline, 13.1 g (94.82 mmol) of potassium carbonate, and 1.8 g (1.58 mmol) of tetrakis(triphenylphosphine)palladium were added to a round-bottomed flask and stirred overnight while performing reflux in 140 mL of 1,4-dioxane and 70 mL of water. Upon completion of the reaction, the mixture was cooled to room temperature, and then EtOH was added so that the solid was filtered and washed with EtOH and water. The solid was dissolved in dichlorobenzene, and then filtered under reduced pressure through silica gel and Celite to obtain a filtrate. The obtained filtrate was concentrated under reduced pressure and then recrystallized from dichlorobenzene to obtain 13.2 g (68.2%) of Compound 29.

**Synthetic Example 7. Synthesis of Compound 35**

Synthesis of Intermediate 13

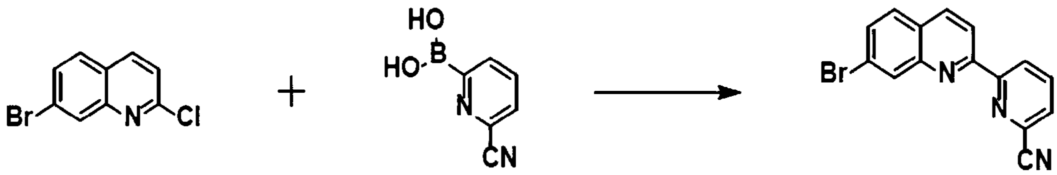

20.0 g (82.47 mmol) of 7-bromo-2-chloroquinoline, 13.4 g (90.72 mmol) of (6-cyanopyridin-2-yl)boronic acid, 34.2 g (247.42 mmol) of potassium carbonate, and 4.8 g (4.12 mmol) of tetrakis(triphenylphosphine)palladium were added to a round-bottomed flask and stirred overnight while performing reflux in 200 mL of 1,4-dioxane and 100 mL of water. Upon completion of the reaction, the mixture was cooled to room temperature, and then EtOH was added so that the solid was filtered and washed with EtOH and water. The solid was dissolved in toluene, and then filtered under reduced pressure through silica gel and Celite to obtain a filtrate. The obtained filtrate was concentrated under reduced pressure and then recrystallized from toluene to obtain 18.6 g (72.7%) of Intermediate 13.

Synthesis of Intermediate 14

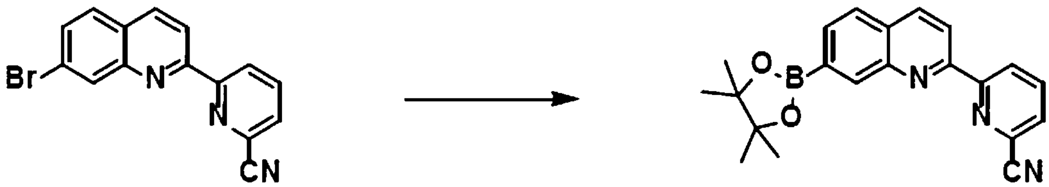

18.5 g (59.65 mmol) of Intermediate 13, 18.2 g (71.58 mmol) of bis(pinacolato)diboron, 17.6 g (178.95 mmol) of potassium acetate, and 1.5 g (1.79 mmol) of [1,1'-Bis(diphenylphosphino)ferrocene]dichloropalladium(II) were added to a round-bottomed flask and stirred overnight while performing reflux in 250 mL of 1,4-dioxane. Upon completion of the reaction, the mixture was cooled to room temperature, and then concentrated under reduced pressure to obtain a filtrate. The obtained filtrate was extracted using ethyl acetate and water. The organic material layer was dehydrated with $MgSO_4$ and then concentrated under reduced pressure to obtain a filtrate. The obtained filtrate was dissolved in dichloromethane, filtered through silica gel and Celite under reduced pressure, and concentrated under reduced pressure to obtain 16.4 g

(77.0%) of Intermediate 14.

Synthesis of Intermediate 15

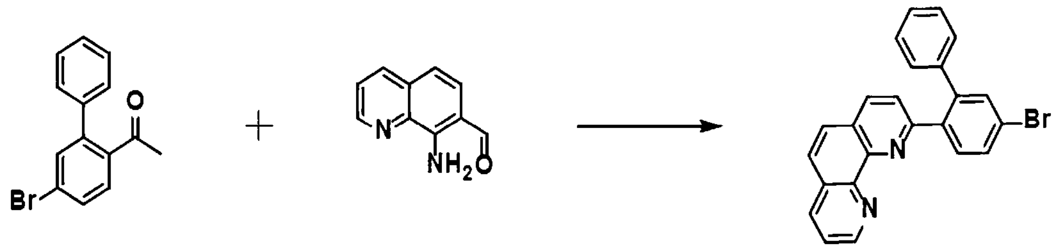

20.0 g (72.69 mmol) of 1-(5-bromo-[1,1'-biphenyl]-2-yl)ethan-1-one, 16.3 g (94.49 mmol) of 8-aminoquinoline-7-carbaldehyde, and 140 mL of toluene were added to a round-bottomed flask. After dissolving 12.2 g (218.06 mmol) of potassium hydroxide was dissolved in 100 mL of ethanol, potassium hydroxide dissolved in ethanol was added dropwise, and the mixture was stirred at 80°C overnight. Upon completion of the reaction, after the mixture was cooled to room temperature, methanol was added thereto to filter the mixture, and the solid was washed with methanol. The solid was dissolved in toluene, and then filtered under reduced pressure through silica gel and Celite to obtain a filtrate. The obtained filtrate was concentrated under reduced pressure and then recrystallized from toluene to obtain 16.1 g (61.3%) of Intermediate 15.

Synthesis of Compound 35

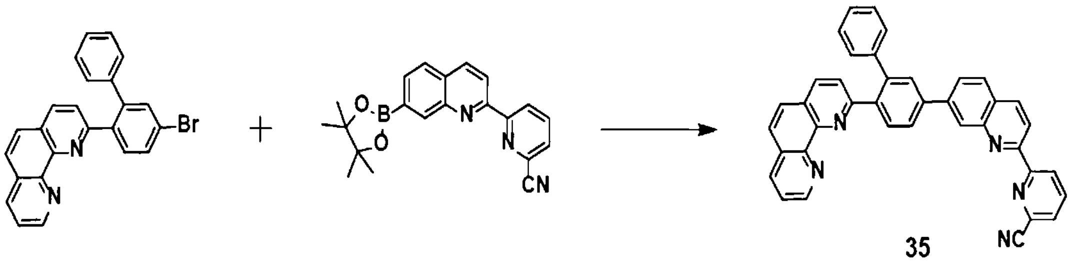

35

16.0 g (44.29 mmol) of Intermediate 15, 17.4 g (48.72 mmol) of Intermediate 14, 18.4 g (132.88 mmol) of potassium carbonate, and 2.6 g (2.21 mmol) of tetrakis(triphenylphosphine)palladium were added to a round-bottomed flask and stirred overnight while performing reflux in 160 mL of 1,4-dioxane and 80 mL of water. Upon completion of the reaction, the mixture was cooled to room temperature, and then EtOH was added so that the solid was filtered and washed with EtOH and water. The solid was dissolved in dichlorobenzene, and then filtered under reduced pressure through silica gel and Celite to obtain a filtrate. The obtained filtrate was concentrated under reduced pressure and then recrystallized from dichlorobenzene to obtain 15.7 g (63.1%) of Compound 35.

**Synthetic Example 8. Synthesis of Compound 53**

Synthesis of Intermediate 16

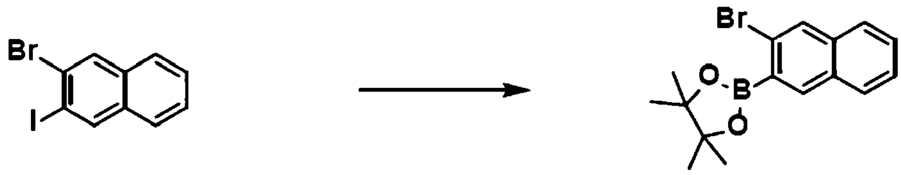

15.0 g (45.05 mmol) of 2-bromo-3-iodonaphthalene, 13.7 g (54.06 mmol) of bis(pinacolato)diboron, 13.3 g (135.15 mmol) of potassium acetate, and 1.1 g (1.35 mmol) of [1,1'Bis(diphenylphosphino)ferrocene]dichloropalladium(II) were added to a round-bottomed flask and stirred overnight while performing reflux in 225 mL of 1,4-dioxane. Upon completion of the reaction, the mixture was cooled to room temperature, and then concentrated under reduced pressure to obtain a filtrate. The obtained filtrate was extracted using ethyl acetate and water. The organic material layer was dehydrated with $MgSO_4$ and then concentrated under reduced pressure to obtain a filtrate. The obtained filtrate was dissolved in dichloromethane, filtered through silica gel and Celite under reduced pressure, and concentrated under reduced pressure to obtain 12.7 g (84.7%) of Intermediate 16.

Synthesis of Intermediate 17

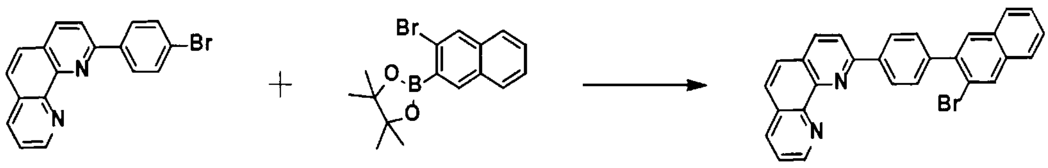

11.0 g (32.82 mmol) of 2-(4-bromophenyl)-1,10-phenanthroline, 12.0 g (36.10 mmol) of Intermediate 16, 13.6 g (98.45 mmol) of potassium carbonate, and 1.9 g (1.64 mmol) of tetrakis(triphenylphosphine)palladium were added to a round-bottomed flask and stirred overnight while performing reflux in 110 mL of 1,4-dioxane and 55 mL of water. Upon completion of the reaction, the mixture was cooled to room temperature, and then EtOH was added so that the solid was filtered and washed with EtOH and water. The solid was dissolved in toluene, and then filtered under reduced pressure through silica gel and Celite to obtain a filtrate. The obtained filtrate was concentrated under reduced pressure and then recrystallized from toluene to obtain 9.4 g (62.1%) of Intermediate 17.

Synthesis of Compound 53

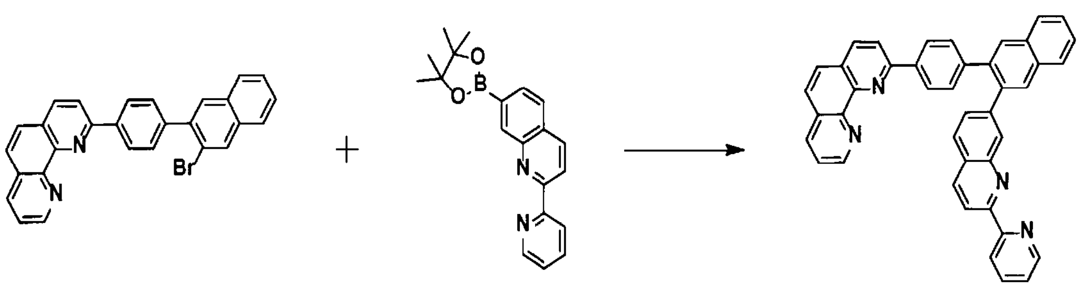

53

9.0 g (19.51 mmol) of Intermediate 17 and 7.1 g (21.46 mmol) of 2-(pyridin-2-yl)-7-(4,4,5,5-tetramethyl-1,3,2-dioxaborolan-2-yl)quinoline, 8.1 g (58.52 mmol) of potassium carbonate, and 1.1 g (0.98 mmol) of tetrakis(triphenylphosphine)palladium were added to a round-bottomed flask and stirred overnight while performing reflux in 90 mL of 1,4-dioxane and 45 mL of water. Upon completion of the reaction, the mixture was cooled to room temperature, and then EtOH was added so that the solid was filtered and washed with EtOH and water. The solid was dissolved in dichlorobenzene, and then filtered under reduced pressure through silica gel and Celite to obtain a filtrate. The obtained filtrate was concentrated under reduced pressure and then recrystallized from dichlorobenzene to obtain 8.3 g (72.5%) of Compound 53.

**Experimental Example 1. Measurement of RE Values and $k_{et}(e)/k_{et}(h)$ Values**

The reorganization energy (RE) values and the movement rate constant ratios of holes and electrons ($k_{et}(e)/K_{et}(h)$) of the previous compounds of the structures synthesized in Synthetic Examples 1 to 8 and Comparative Compounds 1 to 5 shown in Table 1 below were measured respectively. Specifically, the RE values and $k_{et}(e)/k_{et}(h)$ values were calculated at the DFT b3lyp/6-31g(d) level using the Gaussian 09W program. The results are shown in Table 2 below.

[Table 1]

| | |
|---|---|
| 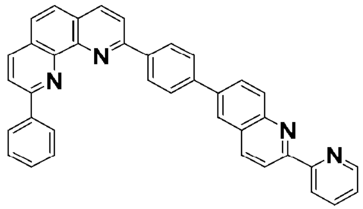 | 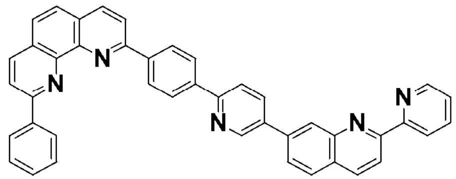 |
| Comparative Compound 1 | Comparative Compound 2 |

| | | |
|---|---|---|
| 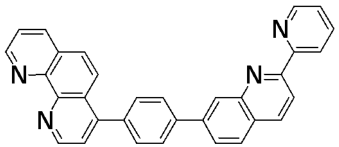 | 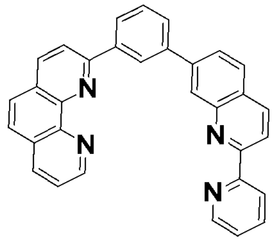 | 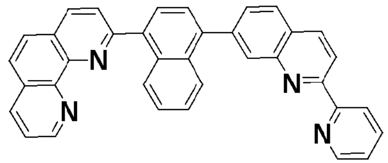 |
| Comparative Compound 3 | Comparative Compound 4 | Comparative Compound 5 |

[Table 2]

| Classification | RE value [eV] | $k_{et}(e)/k_{et}(h)$ value |
|---|---|---|
| Comparative Compound 1 | 0.265 | 0.434 |
| Comparative Compound 2 | 0.158 | 0.883 |
| Comparative Compound 3 | 0.299 | 0.603 |
| Comparative Compound 4 | 0.175 | 2.414 |
| Comparative Compound 5 | 0.276 | 1.341 |
| Compound 1 | 0.218 | 1.198 |
| Compound 3 | 0.162 | 1.076 |
| Compound 6 | 0.140 | 1.397 |
| Compound 16 | 0.137 | 1.400 |
| Compound 20 | 0.115 | 1.097 |
| Compound 29 | 0.183 | 1.194 |
| Compound 35 | 0.195 | 2.485 |
| Compound 53 | 0.153 | 2.799 |

As shown in Table 2 above, it could be seen that the RE values of Compounds of the present disclosure are relatively low compared to Comparative Compounds, and at the same time, it could be confirmed that the $k_{et}(e)/k_{et}(h)$ values are relatively high. As such, in the case of Compounds of the present disclosure, since the RE values are relatively low so that charge loss is small during the charge recombination process, it is expected that the movement of electrons can be made smoothly and electrons can be effectively transferred between molecules, and as a result, it can be expected that Compounds of the present disclosure are effective in terms of improving the efficiency of the device. At the same time, since Compounds of the present disclosure have relatively high $k_{et}(e)/k_{et}(h)$ values so that the electron movement speed is fast, the electron movement is smooth so that it can be expected that Compounds of the present disclosure are also effective in terms of improving the efficiency of the device. On the other hand, although Comparative Compound 2 satisfies the low RE value, the ket(e)/ket(h) value is also low, so it is expected that the improvement in the efficiency of the device will be insignificant, which is not desirable. In addition, it could be confirmed that Comparative Compound 4 had a low RE value and a high $k_{et}(e)/k_{et}(h)$ value at the levels of Compounds of the present disclosure. In the case of Comparative Compound 4, as can be seen through Experimental Example 2 conducted afterward using a compound that uses only 1,3-phenylene as a linker, it is not desirable for use in device manufacturing in that since its thermal stability is weak, its shape can be easily deformed by heat or thermal decomposition can be easily promoted. In addition, Comparative Compound 5 has a high RE value, which is not desirable in terms of improving device efficiency, and as can be seen through Experimental Example 2 conducted afterward, its thermal stability is also weak, which is not desirable.

**Experimental Example 2. Thermal Stability Evaluation**

In order to evaluate the thermal stability using previously synthesized Compounds 1 and 3 and Comparative Compound 4 of Table 1 above as representatives, the glass transition temperatures (Tg) and decomposition temperatures (Td) were respectively measured. The results are shown as in Table 3-1 below.

[Table 3-1]

| Classification | Structure | Glass transition temperature (Tg) [°C] | Decomposition temperature (Td, 5%) [°C] |
|---|---|---|---|
| Comparative Compound 4 | | 108.42 | 396.83 |
| Compound 1 | 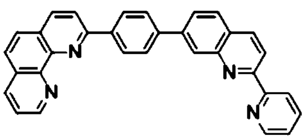 | 113.67 | 412.27 |

(continued)

| Classification | Structure | Glass transition temperature (Tg) [°C] | Decomposition temperature (Td, 5%) [°C] |
|---|---|---|---|
| Compound 3 | 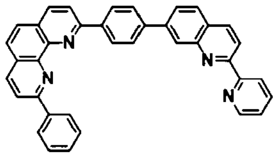 | 124.15 | 447.86 |

As shown in Table 3-1, it could be confirmed that Comparative Compound 4 had lower Tg and also had lower Td than Compounds of the present disclosure. It could be confirmed through this that 1,3-phenylene is more vulnerable in terms of thermal stability than 1,4-phenylene. Meanwhile, it could be confirmed that Compound 3 of the present disclosure had excellent thermal stability compared to Compound 1 of the present disclosure, and through this, it could be confirmed that the phenyl group bonded to $R_1$, immediately adjacent to 1,10-phenanthroline N, influences on the improvement of thermal stability.

In addition, in order to evaluate the thermal stability of the previously synthesized Compound 1 and Compound 3 of the present disclosure and Comparative Compound 5 of Table 1 as representatives, the purity was measured at +30°C based on the sublimation temperature (Ts) of each compound. The results are shown as in Table 3-2 below.

[Table 3-2]

| Classification | Structure | Purity (sublimation temperature+30°C) |
|---|---|---|
| Comparative Compound 5 | | 81.23% |
| Compound 1 | 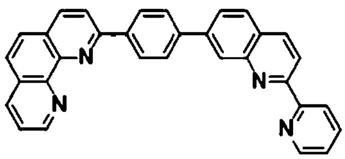 | 93.44% |
| Compound 3 | 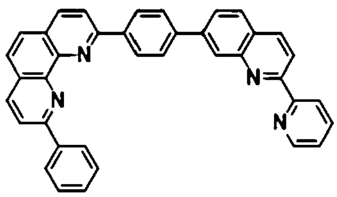 | 99.97% |

As shown in Table 3-2, it could be confirmed that in the case of Compounds of the present disclosure, purities of 90% or more were maintained, whereas in the case of Comparative Compound, thermal decomposition was significantly progressed with a purity of approximately 81%. Meanwhile, as in the results in Table 3-1, it can be confirmed that Compound 3 of the present disclosure had more excellent thermal stability than that of Compound 1 of the present disclosure.

**Experimental Example 3. Fabrication and Evaluation of Organic light emitting devices - Use of Li Dopant**

Comparative Example 1

An ITO substrate, which became the anode, was patterned so that the emission area became a 2 mm × 2 mm size, and then cleaned with isopropyl alcohol and UV ozone, respectively. Thereafter, the ITO substrate was mounted on a substrate holder in a vacuum deposition device, and the pressure was maintained so that the vacuum degree became $1\times10^{-7}$ torr. Plasma treatment was then performed for 3 minutes under a $N_2$ atmosphere. First, the HAT-CN compound was vacuum-deposited to form a first hole injection layer with a thickness of 5 nm. An NPB material was vacuum-deposited thereon to form a first hole transport layer with a thickness of 20 nm. Thereafter, a green first emission layer with a thickness of 20 nm was formed by performing co-deposition using a GH-1 material as a host and a GD-1 material as a dopant so that it had a mass ratio of about 10%. A TmPyPb compound was vacuum-deposited on this first emission layer to form a first electron transport layer with a thickness of 20 nm. Thereafter, an N-type charge generation layer with a thickness of 10 nm

was formed by performing co-deposition using a BPhen material as a host and Li as a dopant so that it had a mass ratio of 2%. Thereafter, the HAT-CN compound was vacuum-deposited to form a P-type charge generation layer with a thickness of 5 nm. This P-type charge generation layer is also used as a second hole injection layer. The NPB material was vacuum-deposited thereon to form a second hole transport layer with a thickness of 50 nm. Afterwards, a green second emission layer with a thickness of 20 nm was formed by performing co-deposition using a GH-1 material as a host and a GD-1 material as a dopant so that it had a mass ratio of about 10%. A second electron transport layer with a thickness of 20 nm was formed on this emission layer by performing co-deposition using a TmPyPb material as a host and an Liq material as a dopant so that it had a mass ratio of about 33%. Thereafter, an LiF material was vacuum-deposited to form an electron injection layer with a thickness of 1 nm. Finally, aluminum (Al) was deposited at a thickness of 50 nm to form a cathode, thereby fabricating the final tandem-type organic light emitting device. The structures of the previously used HAT-CN, NPB, GH-1, GD-1, TmPyPb, BPhen, and Liq materials are respectively as in Table 4 below.

[Table 4]

| | | |
|---|---|---|
| 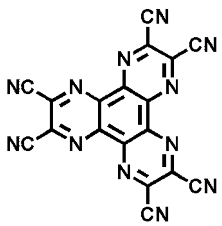 | 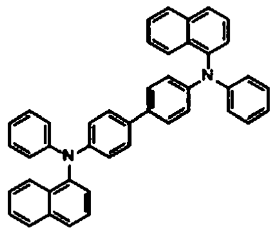 | |
| HAT-CN | NPB | GH-1 |
| 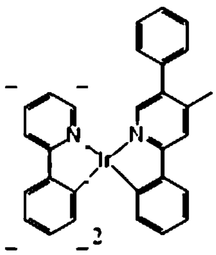 | 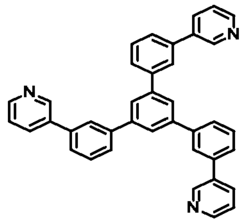 | 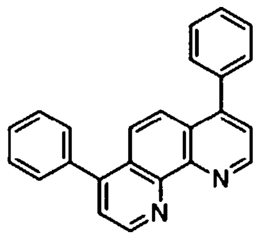 |
| GD-1 | TmPyPb | BPhen |
| | | |
| Liq | | |

Comparative Examples 2 to 6

Organic light emitting devices were fabricated in the same manner as in Comparative Example 1 except that Comparative Compounds 1 to 5 in Table 1 above were respectively used instead of BPhen as the N-type charge generation layer host.

**Examples 1 to 8**

Organic light emitting devices were fabricated in the same manner as in Comparative Example 1 except that compounds of the present disclosure synthesized in previous Synthetic Examples 1 to 8 were respectively used instead of BPhen as the N-type charge generation layer host.

The driving voltages, current efficiencies, and lifespans of the previously fabricated organic light emitting devices were respectively evaluated. The evaluation results are as in Table 5 below.

[Table 5]

| Classificatio n | N-type charge generation layer | | Current density (mA/cm$^2$) | Driving voltage(V) | Current efficiency (cd/A) | Lifespan (T95) |
|---|---|---|---|---|---|---|
| | Host | Dopant | | | | |
| Comparative Example 1 | BPhen | Li | 10 | 6.81 | 117.41 | 184.07 |

(continued)

| Classificatio n | N-type charge generation layer | | Current density ($mA/cm^2$) | Driving voltage(V) | Current efficiency (cd/A) | Lifespan (T95) |
|---|---|---|---|---|---|---|
| | Host | Dopant | | | | |
| Comparative Example 2 | Comparative Compound 1 | Li | 10 | 6.77 | 117.73 | 181.93 |
| Comparative Example 3 | Comparative Compound 2 | Li | 10 | 6.85 | 118.59 | 190.67 |
| Comparative Example 4 | Comparative Compound 3 | Li | 10 | 6.75 | 117.57 | 183.93 |
| Comparative Example 5 | Comparative Compound 4 | Li | 10 | 6.66 | 118.69 | 188.27 |
| Comparative Example 6 | Comparative Compound 5 | Li | 10 | 6.70 | 118.53 | 186.57 |
| Example 1 | Compound 1 | Li | 10 | 6.58 | 120.47 | 204.07 |
| Example 2 | Compound 3 | Li | 10 | 6.64 | 122.68 | 209.33 |
| Example 3 | Compound 6 | Li | 10 | 6.53 | 120.29 | 201.47 |
| Example 4 | Compound 16 | Li | 10 | 6.42 | 120.15 | 215.40 |
| Example 5 | Compound 20 | Li | 10 | 6.63 | 123.57 | 200.93 |
| Example 6 | Compound 29 | Li | 10 | 6.59 | 122.41 | 207.33 |
| Example 7 | Compound 35 | Li | 10 | 6.62 | 118.92 | 199.47 |
| Example 8 | Compound 53 | Li | 10 | 6.43 | 120.93 | 200.53 |

As shown in Table 5, it could be confirmed that the organic light emitting devices using Compounds of the present disclosure had low driving voltages and improved efficiencies and lifespans. Comparative Compound 1 differs from Compounds of the present disclosure in that 1,10-phenanthroline is bonded to the 6-position of quinoline, Comparative Compound 2 differs from Compounds of the present disclosure in that it includes a heteroarylene group as a linker, and Comparative Compound 3 differs from Compounds of the present disclosure in that the quinoline-pyridine is bonded to a position other than the position immediately next to N of 1,10-phenanthroline. Due to these differences, it can be interpreted that Comparative Compounds 1 to 3 had relatively high RE values and low $k_{et}(e)/k_{et}(h)$ values, thereby, as a result, having results of low efficiencies and also having reduced lifespans in device evaluation. On the other hand, when a heteroarylene group is included as a linker as in Comparative Compound 2, it has a relatively too deep LUMO, thereby having a result of a higher driving voltage also. Meanwhile, it could be confirmed that Comparative Compound 4, which differs from Compounds of the present disclosure in that it only uses 1,3-phenylene as a linker, also had poor operating driving voltage, efficiency, and lifetime. It could be confirmed that Comparative Compound 5 also had low driving voltage, efficiency, and lifespan compared to Compounds of the present disclosure.

On the other hand, Compounds of the present disclosure not only satisfy the low RE values and high $k_{et}(e)/k_{et}(h)$ values desirable for application as N-type charge generation layer host compounds, but also have excellent thermal stability, and due to this, it could be confirmed that low voltage, high efficiency, and long lifespan can be achieved when applied to actual devices. Meanwhile, it could be confirmed that among the examples, Compounds 3, 16, and 29, in which particularly $R_1$ immediately next to N of 1,10-phenanthroline is a phenyl group, had excellent lifespans, and through this, it could be seen that when $R_1$ immediately next to N of 1,10-phenanthroline is a phenyl group, not only thermal stability is excellent, but actual device lifespan is also excellent.

**Experimental Example 4. Fabrication and Evaluation of Organic Light Emitting Devices** - Use of Yb Dopant

**Comparative Example 7**

An organic light emitting device was fabricated in the same manner as Comparative Example 1 except that Yb was used instead of Li as an N-type charge generation layer dopant so that the mass ratio became 1%.

**Comparative Examples 8 to 12**

Organic light emitting devices were fabricated in the same manner as Comparative Example 7 except that Comparative Compounds 1 to 5 in Table 1 above were respectively used instead of BPhen as an N-type charge generation layer host.

Examples 9 to 16

Organic light emitting devices were fabricated in the same manner as Comparative Example 7 except that compounds of the present disclosure synthesized in Synthetic Examples 1 to 8 were respectively used instead of BPhen as the N-type charge generation layer host.

The driving voltages, current efficiencies, and lifespans of the previously fabricated organic light emitting devices were respectively evaluated. The evaluation results are as in Table 6 below.

[Table 6]

| Classification | N-type charge generation layer | | Current density ($mA/cm^2$) | Driving voltage (V) | Current efficiency (cd/A) | Lifespan (T95) |
|---|---|---|---|---|---|---|
| | Host | Dopant | | | | |
| Comparative Example 7 | BPhen | Yb | 10 | 7.21 | 114.58 | 136.80 |
| Comparative Example 8 | Comparative Compound 1 | Yb | 10 | 6.85 | 116.00 | 145.00 |
| Comparative Example 9 | Comparative Compound 2 | Yb | 10 | 6.87 | 116.12 | 141.73 |
| Comparative Example 10 | Comparative Compound 3 | Yb | 10 | 6.90 | 115.61 | 139.80 |
| Comparative Example 11 | Comparative Compound 4 | Yb | 10 | 6.97 | 116.51 | 145.07 |
| Comparative Example 12 | Comparative Compound 5 | Yb | 10 | 6.91 | 115.96 | 147.86 |
| Example 9 | Compound 1 | Yb | 10 | 6.50 | 116.97 | 157.47 |
| Example 10 | Compound 3 | Yb | 10 | 6.68 | 120.99 | 161.20 |
| Example 11 | Compound 6 | Yb | 10 | 6.72 | 118.22 | 154.60 |
| Example 12 | Compound 16 | Yb | 10 | 6.73 | 118.23 | 160.33 |
| Example 13 | Compound 20 | Yb | 10 | 6.71 | 121.46 | 149.00 |
| Example 14 | Compound 29 | Yb | 10 | 6.61 | 120.76 | 159.40 |
| Example 15 | Compound 35 | Yb | 10 | 6.74 | 116.56 | 151.07 |
| Example 16 | Compound 53 | Yb | 10 | 6.79 | 118.84 | 153.60 |

As shown in Table 6, also when Yb was used as a dopant, it could be confirmed that similarly to when Li was used, the organic light emitting devices using Compounds of the present disclosure had lower driving voltages and improved efficiencies and lifespans.

Meanwhile, Table 7 below shows the calculated LUMO values of Compounds of the present disclosure synthesized previously. The calculations were performed using the Gaussian 09W program.

[Table 7]

| Classification | LUMO (eV) |
|---|---|
| Compound 1 | -1.80 |
| Compound 3 | -1.84 |

(continued)

| Classification | LUMO (eV) |
|---|---|
| Compound 6 | -1.63 |
| Compound 16 | -1.70 |
| Compound 20 | -1.96 |
| Compound 29 | -1.80 |
| Compound 35 | -2.05 |
| Compound 53 | -1.64 |

When comparing lithium and ytterbium used as dopants in the N-type charge generation layer, the work function of ytterbium is 2.63, which is smaller than the work function of lithium which is 2.93. Therefore, when ytterbium is used, the LUMO of the N-type charge generation layer host must be relatively deep to further facilitate electron movement to the electron transport region (electron transport layer or hole blocking layer), an adjacent layer, and such an influence enables operation even at a low driving voltage. When ytterbium is used according to one embodiment, it is preferable to use a host whose LUMO value satisfies a level of -1.8 eV to -1.9 eV, and as seen from Table 7 above, it can be seen that Compounds 1, 3, and 29 satisfy this. These compounds correspond to compounds in which $L_1$ and $L_2$ are both direct bonds, and which do not contain nitrogen atoms except for 1,10-phenanthroline and quinoline-pyridine. When checking the difference (reduction effect) in driving voltage compared to Comparative Compounds when lithium was used as a dopant and when ytterbium was used as a dopant, respectively, using the results of actual Tables 5 and 6, it could be confirmed in case of Compounds 1, 3, and 29 that the device using the Yb dopant showed a more effective driving voltage reduction effect than other Compounds.

Compounds of the present disclosure described so far include 1,10-phenanthroline, which possesses an unshared pair of electrons, so that Compounds of the present disclosure can readily bind to dopants such as Li and Yb when applied to an N-type charge generation layer. Furthermore, the unshared pair of electrons on nitrogens in quinoline-pyridine more easily helps bonding with dopants so that it is preferable to use Compounds also as hosts for N-type charge generation layers using dopants such as Yb as well as Li. In addition, Compounds of the present disclosure have reorganization energy (RE) values that are low at a certain level or less, and at the same time, satisfy the rate constant ratios of holes and electrons ($k_{et}(e)/k_{et}(h)$) that are a certain level or higher, thereby having effects of increasing the efficiency of the device, etc. as a result. In addition, compounds of the present disclosure have features in which thermal stability is excellent through a structure containing 1,4-phenylene as a linker. When such features enable performance improvements such as low voltage, high efficiency, and long lifespan when applied to an electron transport layer, hole blocking layer, or N-type charge generation layer of an organic light emitting device.

## Claims

**1.** A compound represented by the following Chemical Formula 1:

<Chemical Formula 1>

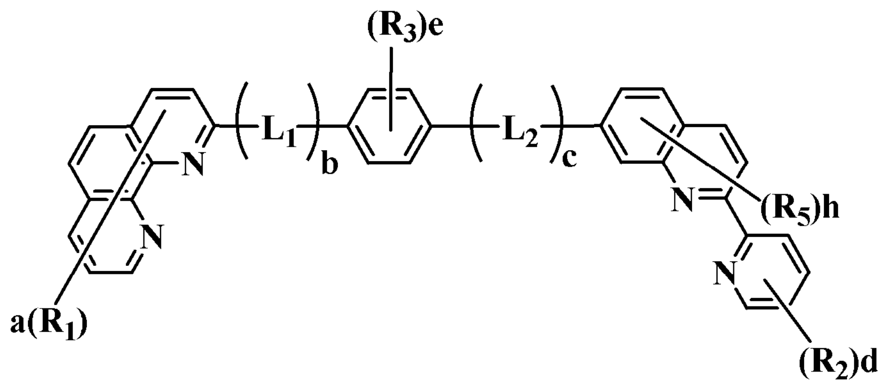

wherein, in Chemical Formula 1,

$R_1$ and $R_2$ are each independently selected from the group consisting of hydrogen, deuterium, a halogen group, a cyano group, a nitro group, a nitrile group, a substituted or unsubstituted $C_1$ to $C_{30}$ alkyl group, a substituted or unsubstituted $C_2$ to $C_{30}$ alkenyl group, a substituted or unsubstituted $C_2$ to $C_{30}$ alkynyl group, a substituted or unsubstituted $C_3$ to $C_{30}$ cycloalkyl group, a substituted or unsubstituted $C_2$ to $C_{30}$ heterocycloalkyl group, a substituted or unsubstituted $C_1$ to $C_{30}$ alkoxy group, a substituted or unsubstituted $C_1$ to $C_{30}$ sulfide group, a substituted or unsubstituted $C_6$ to $C_{30}$ aryl group, a substituted or unsubstituted $C_2$ to $C_{30}$ heteroaryl group, a substituted or unsubstituted $C_6$ to $C_{30}$ aryloxy group, a substituted or unsubstituted $C_2$ to $C_{30}$ heteroaryloxy group, a substituted or unsubstituted $C_1$ to $C_{30}$ thio group, a substituted or unsubstituted $C_1$ to $C_{30}$ amine group, a substituted or unsubstituted $C_1$ to $C_{30}$ silyl group, and a substituted or unsubstituted $C_1$ to $C_{30}$ phosphine oxide group,
$L_1$ and $L_2$ are each independently a direct bond or a substituted or unsubstituted $C_6$ to $C_{30}$ arylene group, and
$R_3$ and $R_5$ are each independently selected from the group consisting of hydrogen, deuterium, - $CD_3$, a halogen group, a cyano group, a nitro group, a nitrile group, a $C_1$ to $C_{30}$ alkyl group, and a $C_6$ to $C_{30}$ aryl group,
a is an integer from 0 to 7,
b and c are each independently an integer from 0 to 2,
d and e are each independently an integer from 0 to 4, and
h is an integer from 0 to 5.

2. The compound of claim 1, wherein $R_1$ and $R_2$ are each independently selected from the group consisting of hydrogen, deuterium, a cyano group, a methyl group, -$CD_3$, a phenyl group, a biphenyl group, a naphthyl group, a pyridine group, a pyrimidine group, a quinoline group, an isoquinoline group, a quinazoline group, and a naphthyridine group, wherein each group is substituted or unsubstituted with one or more substituents selected from the group consisting of deuterium, a cyano group, a methyl group, -$CD_3$, a phenyl group, a naphthyl group, and a pyridine group.

3. The compound of claim **1,** wherein $R_3$ and $R_5$ are each independently selected from the group consisting of hydrogen, deuterium, a cyano group, a methyl group, -$CD_3$, a phenyl group, a biphenyl group, and a naphthyl group, wherein each group is substituted or unsubstituted with one or more substituents selected from the group consisting of deuterium, a cyano group, a methyl group, -$CD_3$, a phenyl group, and a naphthyl group.

4. The compound of claim **1,** wherein $L_1$ and $L_2$ are each independently a direct bond or a $C_6$ to $C_{20}$ arylene group substituted or unsubstituted with one or more substituents selected from the group consisting of deuterium, -$CD_3$, a cyano group, a methyl group, a phenyl group, and a naphthyl group.

5. The compound of claim **4,** wherein the $C_6$ to $C_{20}$ arylene group is selected from the group consisting of a phenylene group, a biphenylene group, and a naphthylene group.

6. The compound of claim 1, wherein Chemical Formula 1 is selected from the group consisting of following Chemical Formulas 2 to 14:

<Chemical Formula 2>

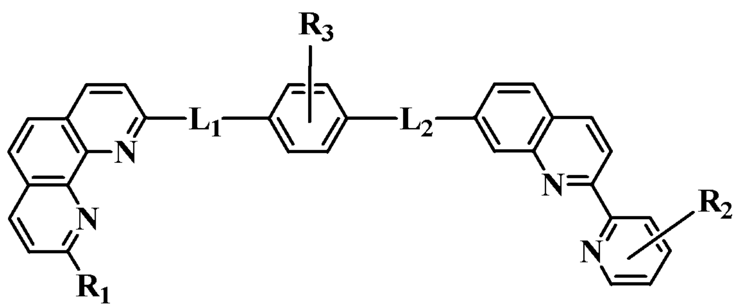

<Chemical Formula 3>

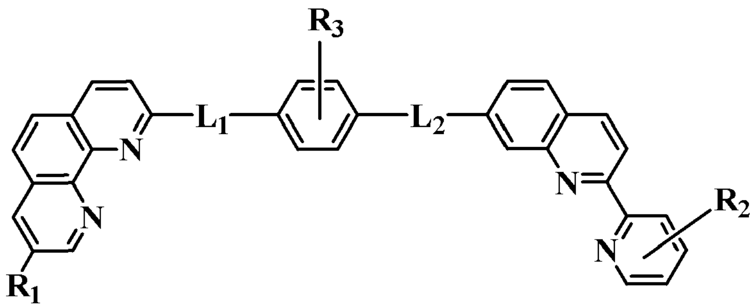

<Chemical Formula 4>

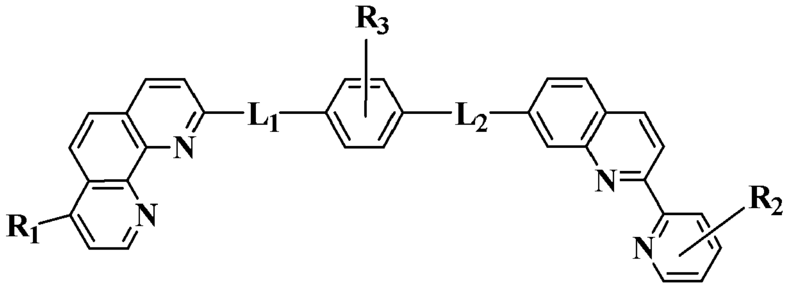

<Chemical Formula 5>

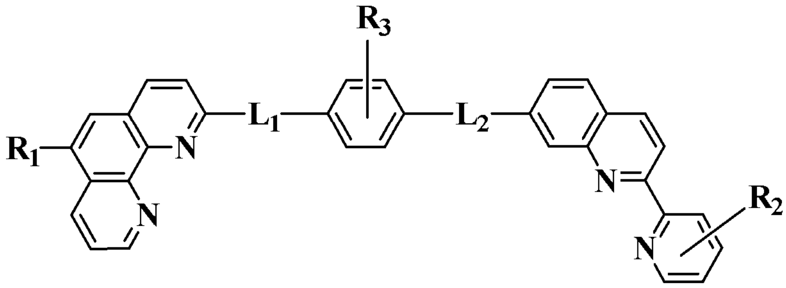

<Chemical Formula 6>

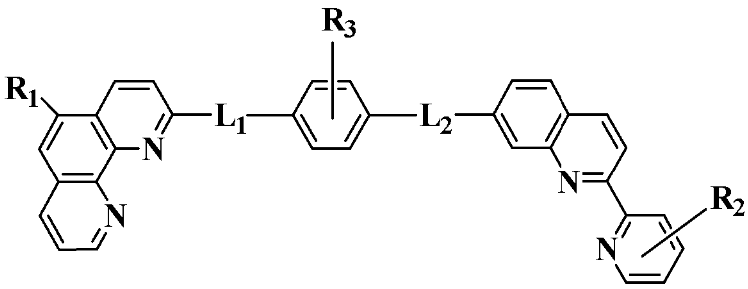

<Chemical Formula 7>

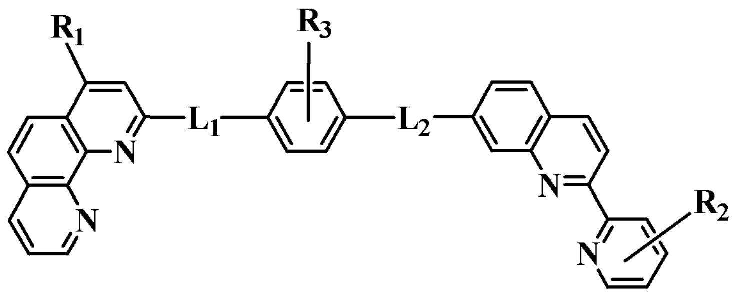

<Chemical Formula 8>

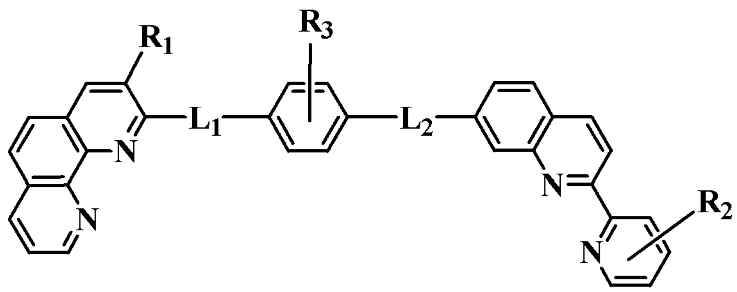

<Chemical Formula 9>

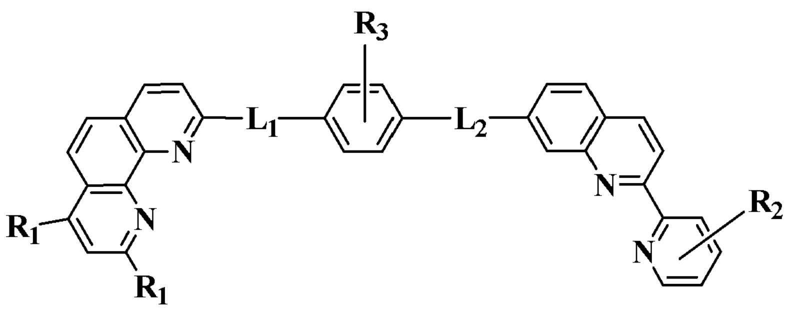

<Chemical Formula 10>

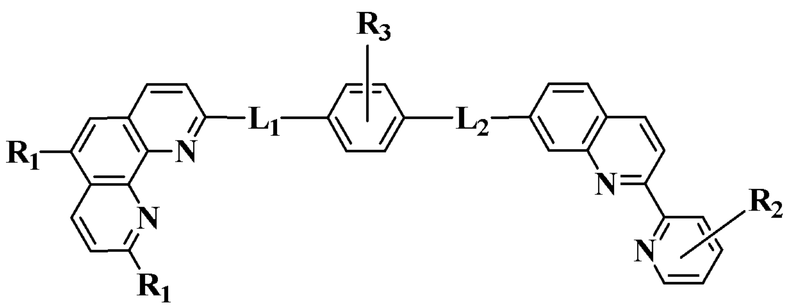

<Chemical Formula 11>

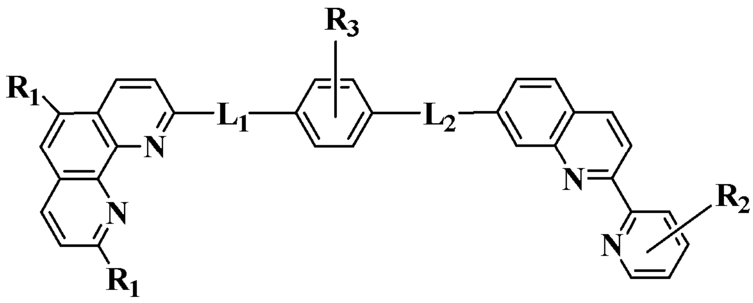

<Chemical Formula 12>

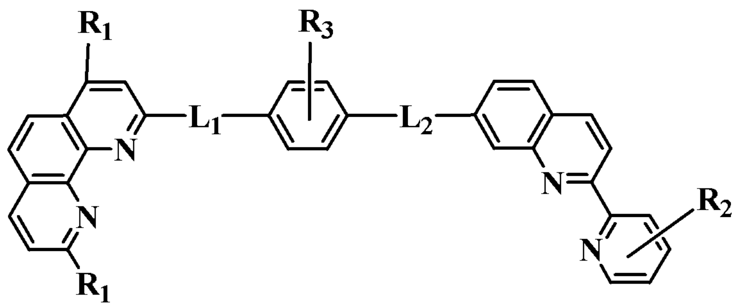

<Chemical Formula 13>

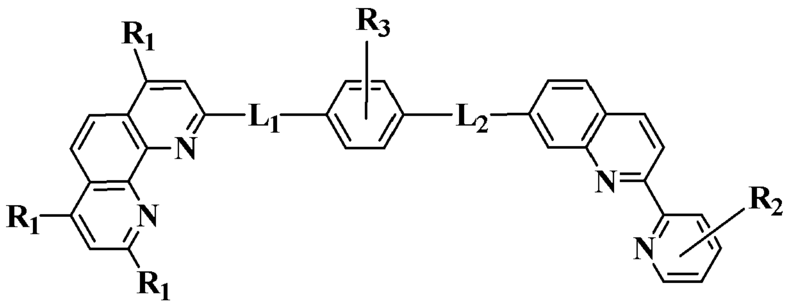

<Chemical Formula 14>

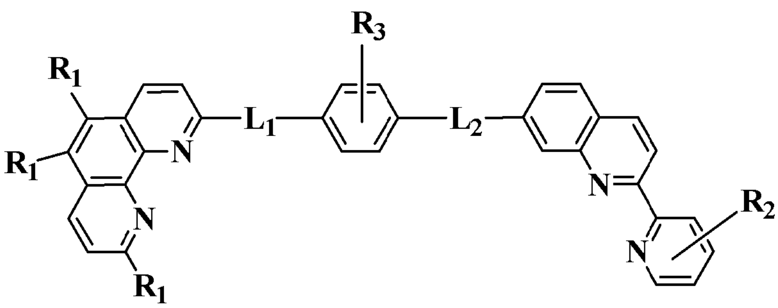

wherein, in Chemical Formulas 2 to 14,

$R_1$ is each independently one of hydrogen, a phenyl group, and a pyridine group,
$R_2$ is each independently one of hydrogen, a cyano group, a phenyl group, and a pyridine group,
$R_3$ is each independently selected from the group consisting of hydrogen, a cyano group, and a phenyl group, and
$L_1$ and $L_2$ are each independently selected from the group consisting of a direct bond, a phenylene group, and a naphthylene group, wherein $L_1$ and $L_2$ are optionally substituted with one of a cyano group and a phenyl group.

**7.** The compound of claim 6, wherein $L_1$ is a direct bond.

8. The compound of claim 6, wherein $R_1$ is hydrogen or a phenyl group

9. The compound of claim 1, wherein the compound is selected from the group consisting of following Compounds 1 to 137:

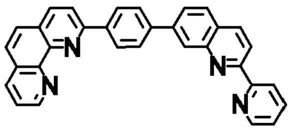

1

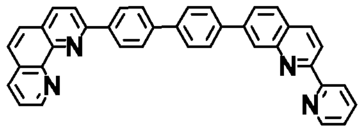

2

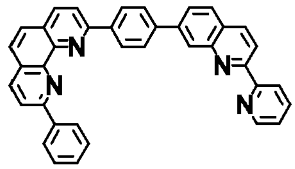

3

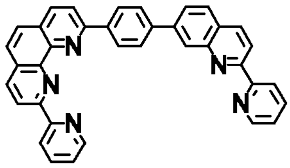

4

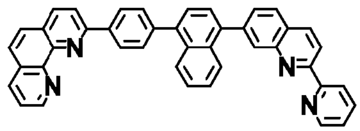

5

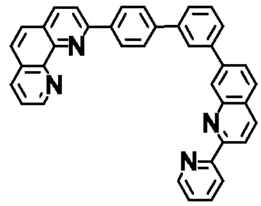

6

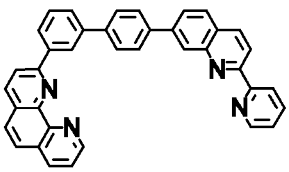

7

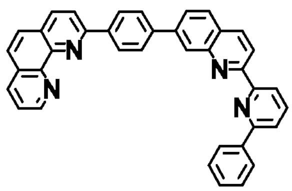

8

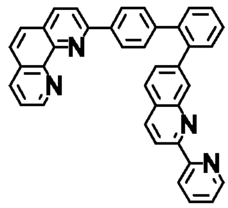

9

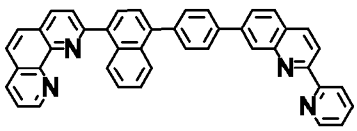

10

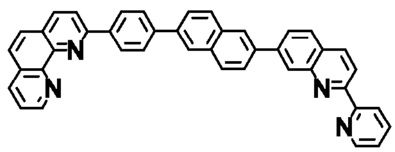

11

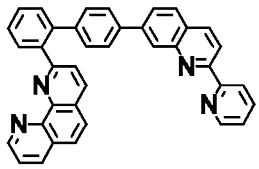

12

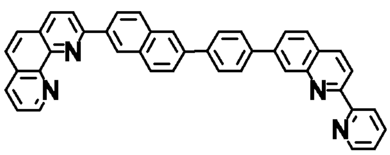

13

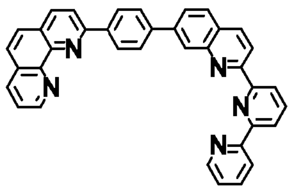

14

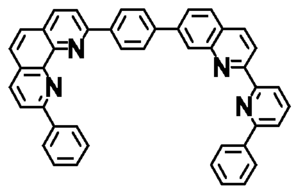

15

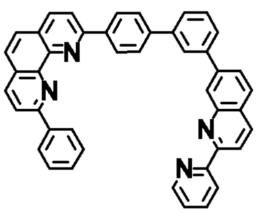

16

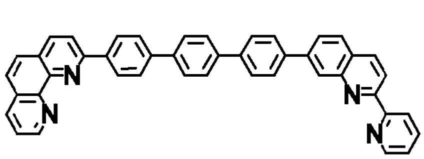

17

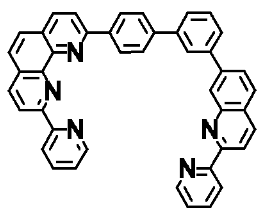

18

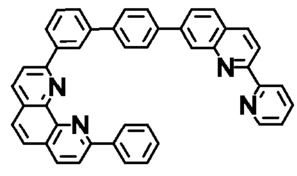

19

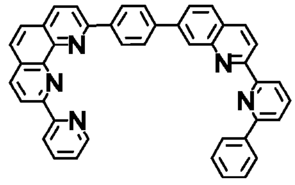

20

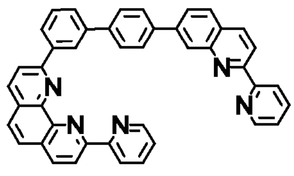

21

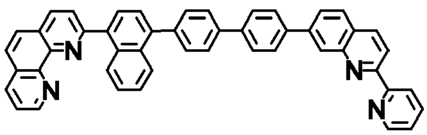

22

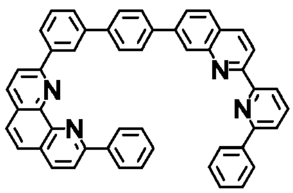

23

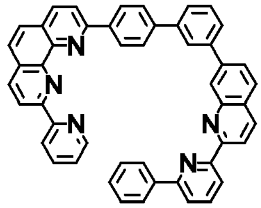

24

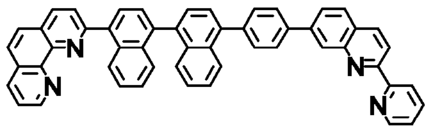

25

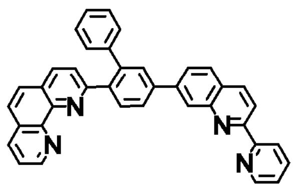

26

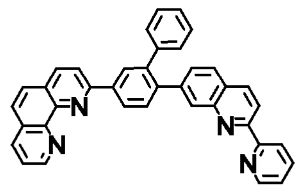

27

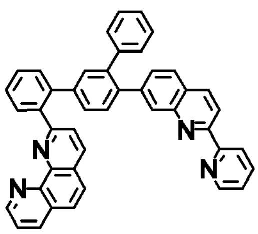

28

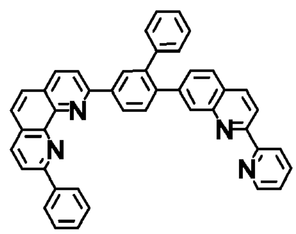

29

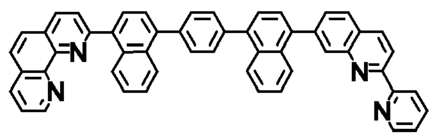

30

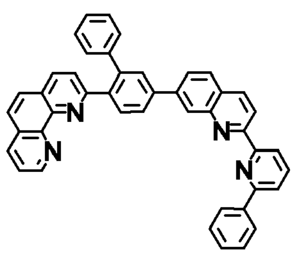

31

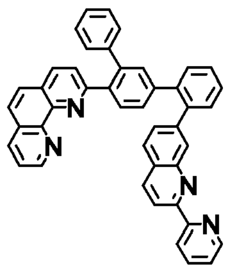

32

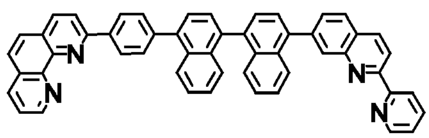

33

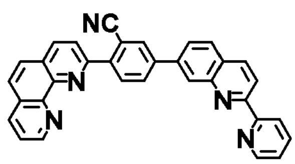

34

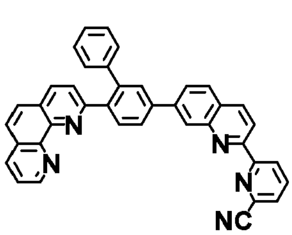

35

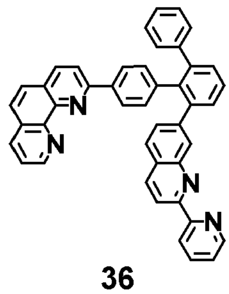

36

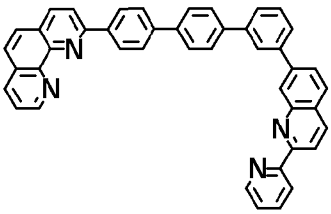
37

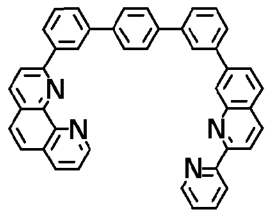
38

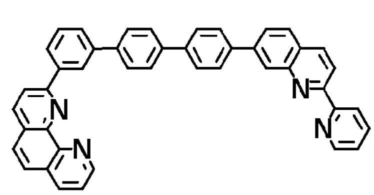
39

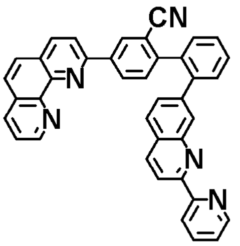
40

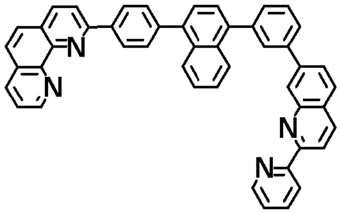
41

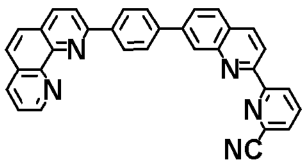
42

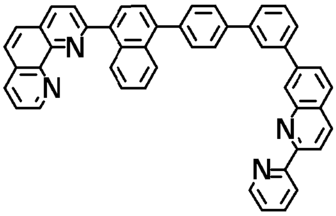
43

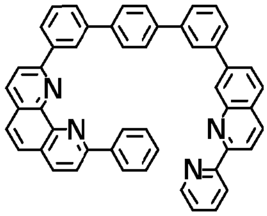
44

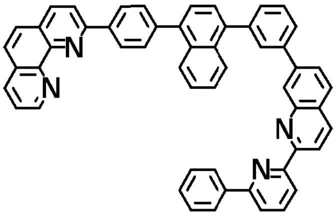
45

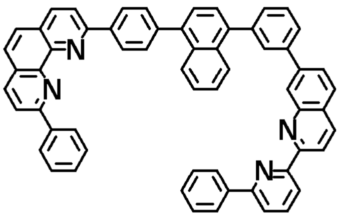
46

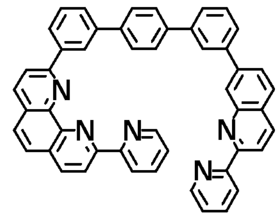
47

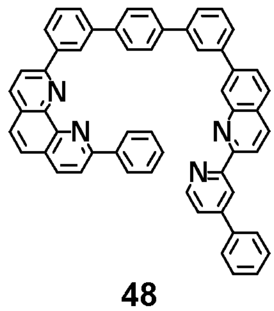
48

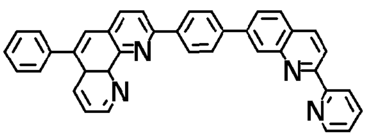
49

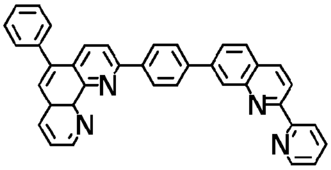
50

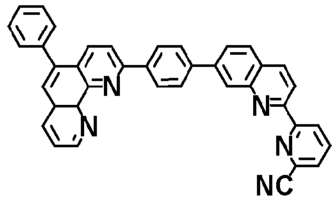
51

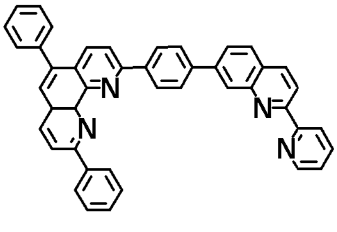
52

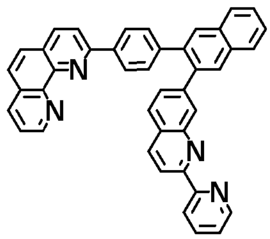
53

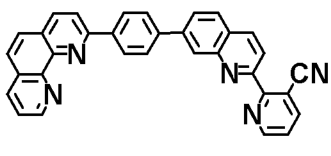
54

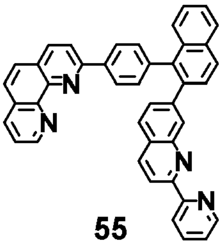
55

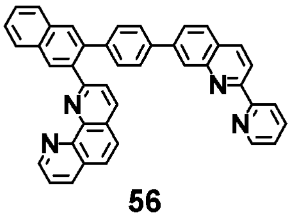
56

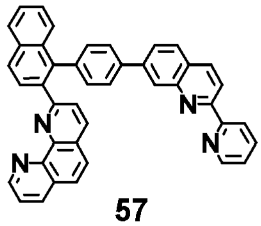
57

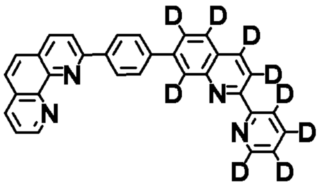
58

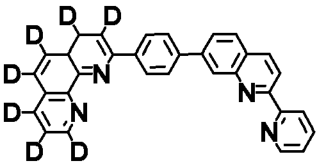
59

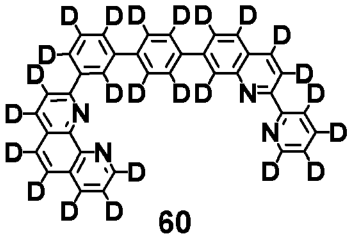
60

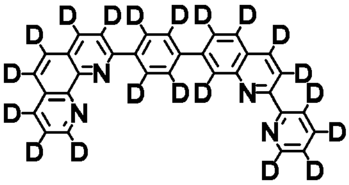
61

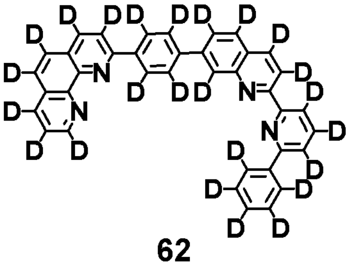
62

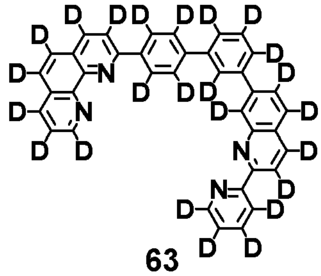
63

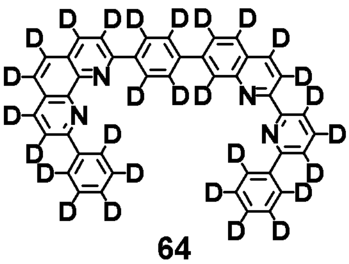
64

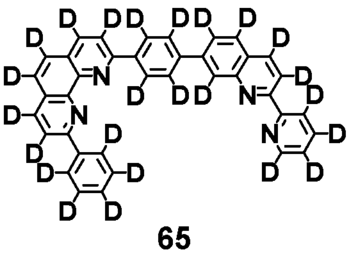
65

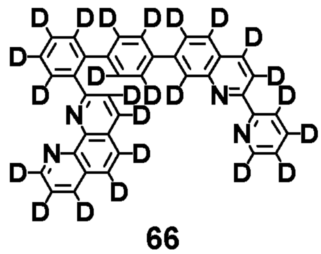
66

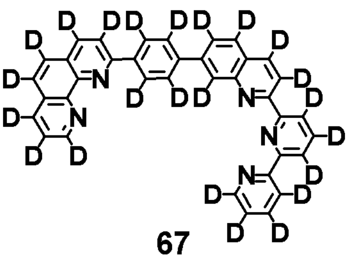
67

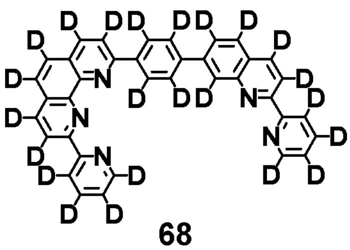
68

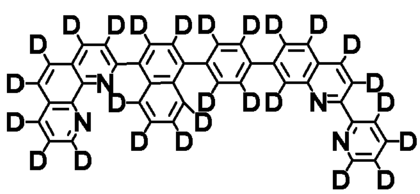
69

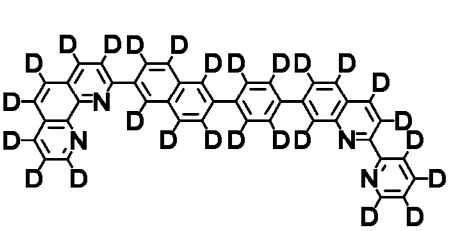
70

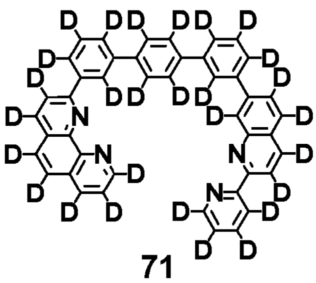
71

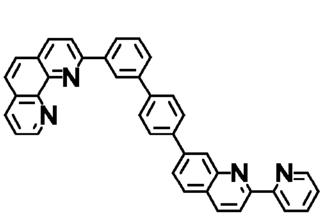
72

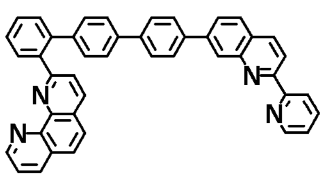

73

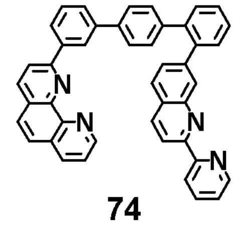

74

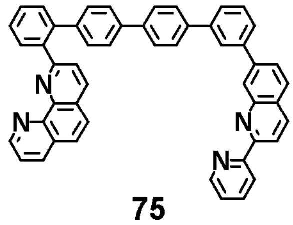

75

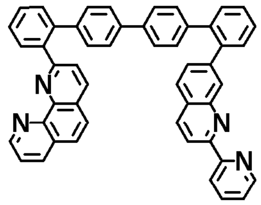

76

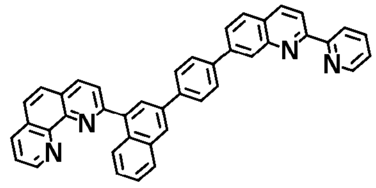

77

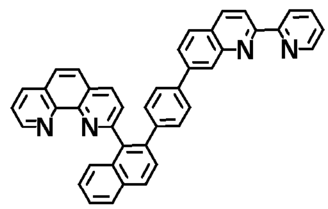

78

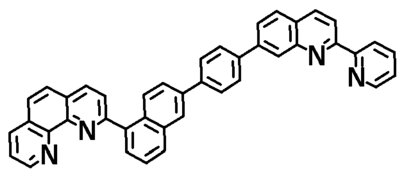

79

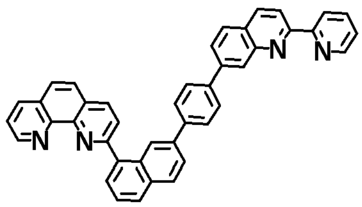

80

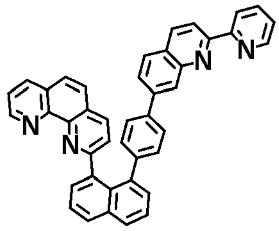

81

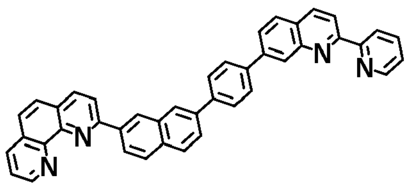

82

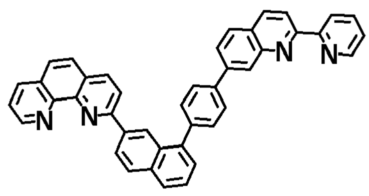

83

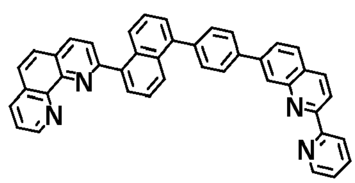

84

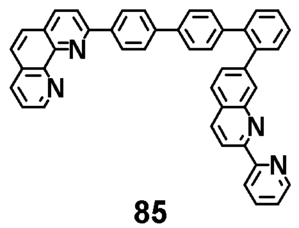

85

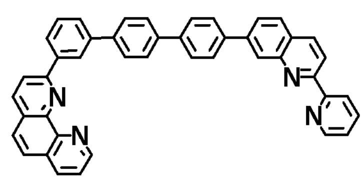

86

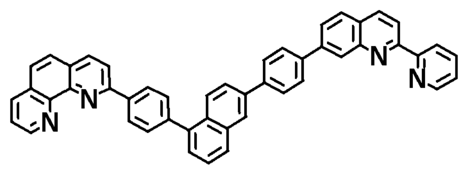

87

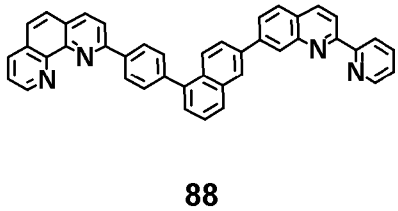

88

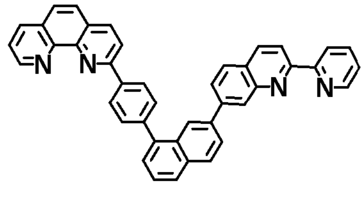

89

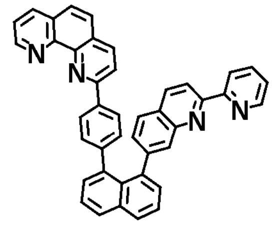

90

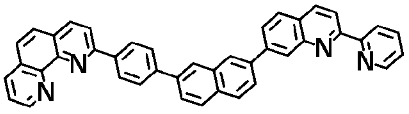

**91**

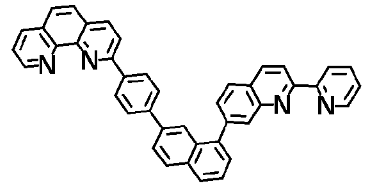

**92**

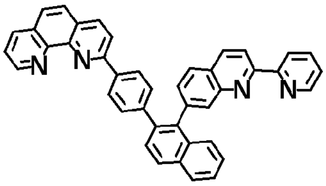

**93**

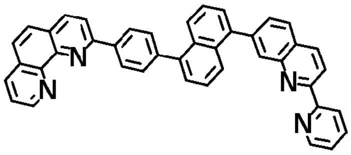

**94**

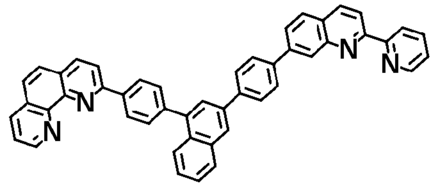

**95**

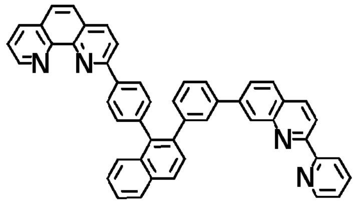

**96**

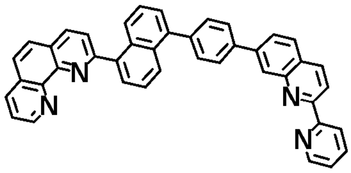

**97**

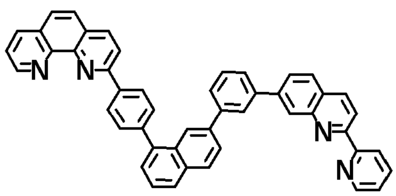

**98**

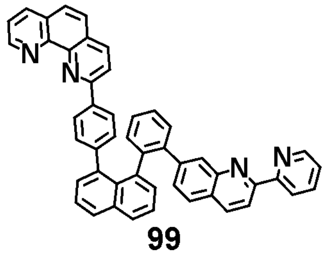

**99**

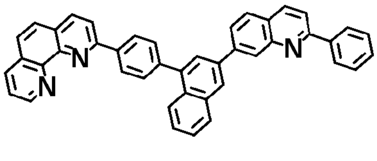

**100**

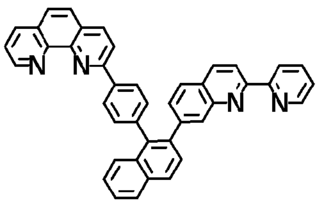

**101**

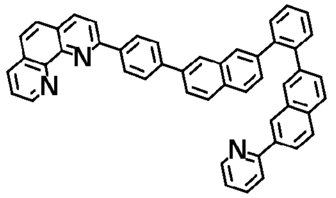

**102**

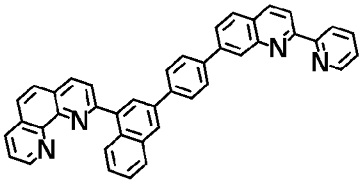

**103**

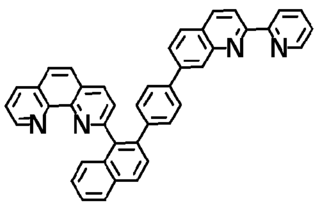

**104**

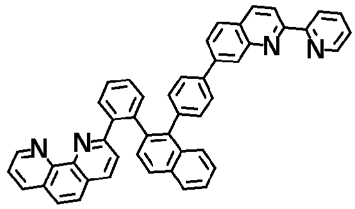

**105**

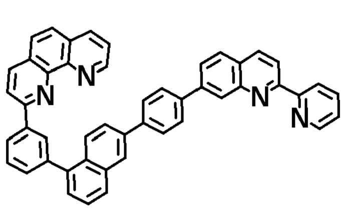

**106**

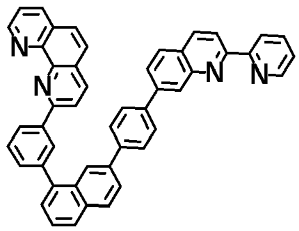

**107**

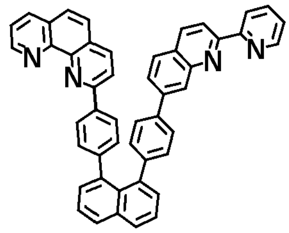

**108**

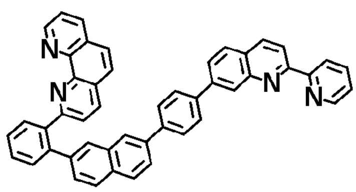

**109**

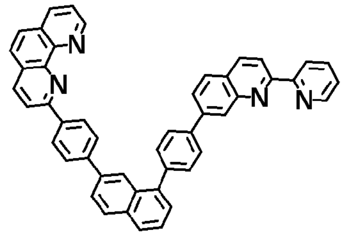

**110**

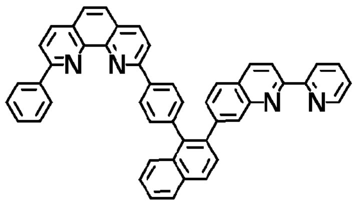

**111**

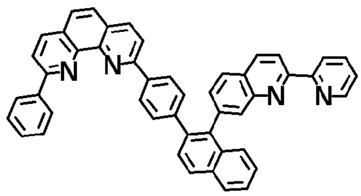

**112**

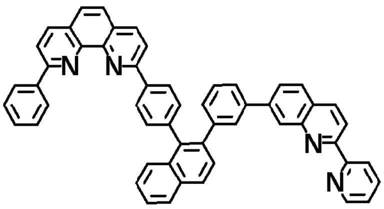

**113**

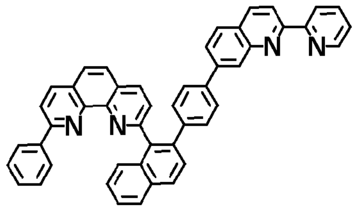

**114**

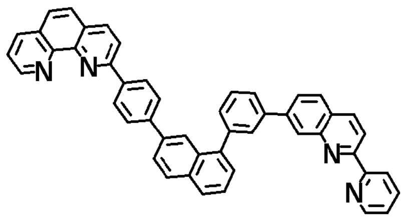

**115**

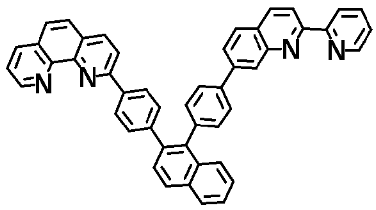

**116**

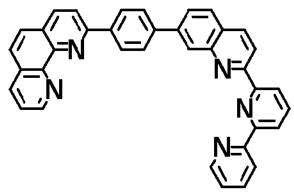

**117**

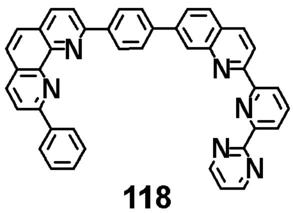

**118**

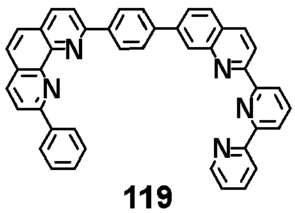

**119**

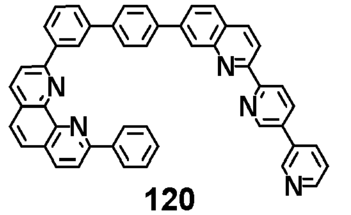

**120**

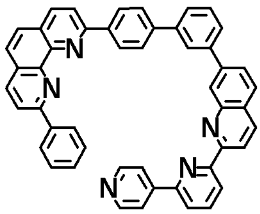

**121**

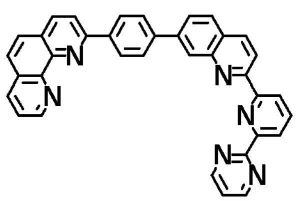

**122**

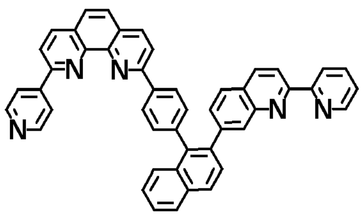

**123**

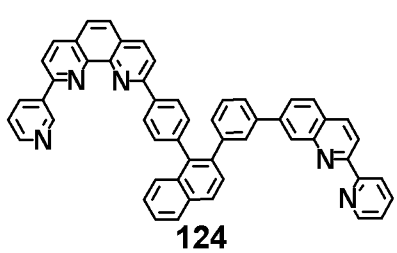

**124**

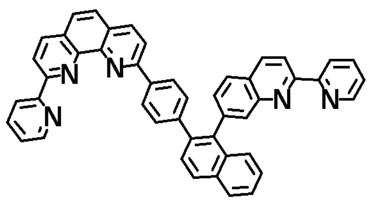

**125**

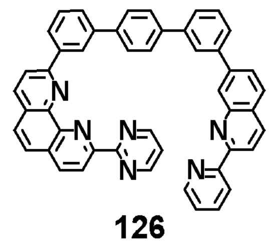

**126**

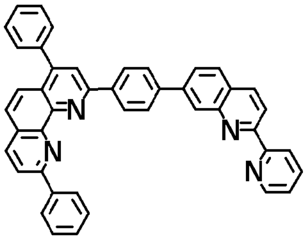

**127**

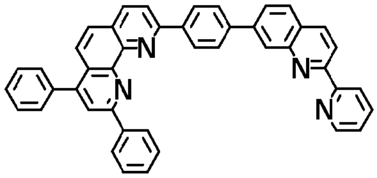

**128**

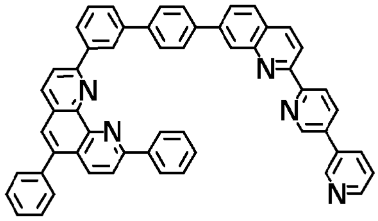

**129**

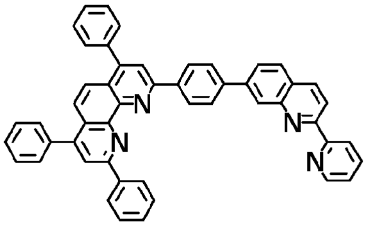

**130**

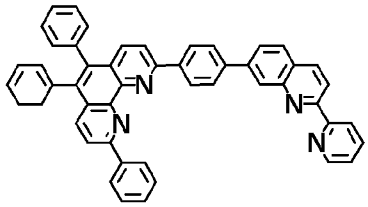

**131**

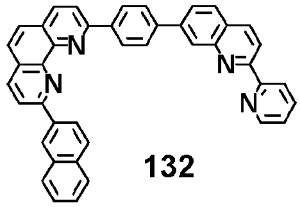

**132**

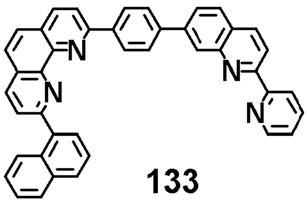

**133**

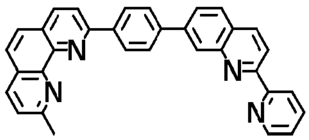

**134**

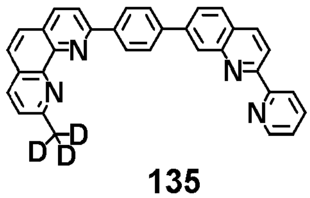

**135**

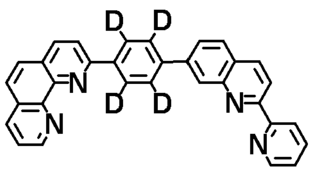

**136**

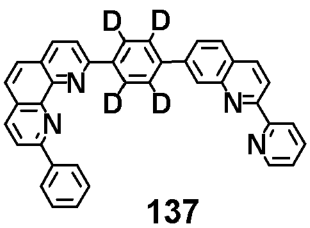

**137**

**10.** An organic light emitting device comprising:

a first electrode and a second electrode; and
one or more organic material layers disposed between the first electrode and the second electrode,
wherein at least one of the organic material layers contains the compound according to any one of claims 1 to 9.

**11.** The organic light emitting device of claim 10, wherein the organic material layer containing the compound is selected from the group consisting of an electron injection layer, an electron transport layer, a hole blocking layer, a layer that simultaneously performs injection and transportation of electrons, and a charge generation layer.

**12.** A tandem-type organic light emitting device comprising:

a first electrode and a second electrode;
a plurality of emission units positioned between the first electrode and the second electrode; and
one or more charge generation layers each positioned at between two adjacent emission units,
wherein at least one charge generation layer includes an N-type charge generation layer and wherein the N-type charge generation layer contains a compound represented by Chemical Formula 1:

<Chemical Formula 1>

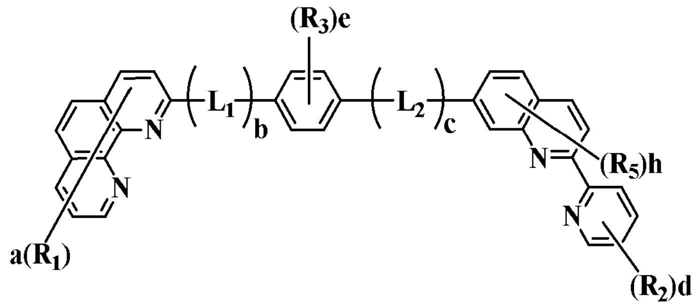

wherein, in Chemical Formula 1,

$R_1$ and $R_2$ are each independently selected from the group consisting of hydrogen, deuterium, a halogen group, a cyano group, a nitro group, a nitrile group, a substituted or unsubstituted $C_1$ to $C_{30}$ alkyl group, a substituted or unsubstituted $C_2$ to $C_{30}$ alkenyl group, a substituted or unsubstituted $C_2$ to $C_{30}$ alkynyl group, a substituted or unsubstituted $C_3$ to $C_{30}$ cycloalkyl group, a substituted or unsubstituted $C_2$ to $C_{30}$ heterocycloalkyl group, a substituted or unsubstituted $C_1$ to $C_{30}$ alkoxy group, a substituted or unsubstituted $C_1$ to $C_{30}$ sulfide group, a substituted or unsubstituted $C_6$ to $C_{30}$ aryl group, a substituted or unsubstituted $C_2$ to $C_{30}$ heteroaryl group, a substituted or unsubstituted $C_6$ to $C_{30}$ aryloxy group, a substituted or unsubstituted $C_2$ to $C_{30}$ heteroaryloxy group, a substituted or unsubstituted $C_1$ to $C_{30}$ thio group, a substituted or unsubstituted $C_1$ to $C_{30}$ amine group, a substituted or unsubstituted $C_1$ to $C_{30}$ silyl group, and a substituted or unsubstituted $C_1$ to $C_{30}$ phosphine oxide group,
$L_1$ and $L_2$ are each independently a direct bond or a substituted or unsubstituted $C_6$ to $C_{30}$ arylene group, and
$R_3$ and $R_5$ are each independently selected from selected from the group consisting of hydrogen, deuterium, $-CD_3$, a halogen group, a cyano group, a nitro group, a nitrile group, a $C_1$ to $C_{30}$ alkyl group, and a $C_6$ to $C_{30}$ aryl group,
a is an integer from 0 to 7,
b and c are each independently an integer from 0 to 2,
d and e are each independently an integer from 0 to 4, and
h is an integer from 0 to 5.

**13.** The tandem-type organic light emitting device of claim 12, wherein the N-type charge generation layer further contains a dopant comprising a metal element, wherein the metal element is selected from the group consisting of lithium (Li), sodium (Na), potassium (K), rubidium (Rb), cesium (Cs), beryllium (Be), magnesium (Mg), calcium (Ca), strontium (Sr), barium (Ba), ytterbium (Yb), samarium (Sm), tin (Sn), copper (Cu), titanium (Ti), cadmium (Cd), mercury (Hg), lead (Pb), bismuth (Bi), zinc (Zn), iron (Fe), cobalt (Co), nickel (Ni), indium (In), gallium (Ga), thorium (Th), uranium (U), silver (Ag), aluminum (Al), gold (Au), molybdenum (Mo), niobium (Nb), palladium (Pd), platinum (Pt), and europium (Eu).

**14.** The tandem-type organic light emitting device of claim 13, wherein when the metal element is ytterbium (Yb), $L_1$ and $L_2$ are direct bonds, and $R_1$ to $R_3$ and $R_5$ do not contain a nitrogen atom.

Europäisches Patentamt
European Patent Office
Office européen des brevets

# EUROPEAN SEARCH REPORT

Application Number
EP 25 21 4112

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | KR 2019 0053579 A (JINWOONG IND CO LTD [KR]) 20 May 2019 (2019-05-20)<br>* claim 4, compound 38 (which is identical with compound 1 of the present application) *<br>* the whole document *<br>----- | 1-4,6-14<br>5 | INV.<br>C09K11/06<br>H10K50/16 |
| A | US 2019/263834 A1 (JEONG WON-JANG [KR] ET AL) 29 August 2019 (2019-08-29)<br>* claim 4, e.g. compound 1-1, 1-20, 2-140 *<br>* the whole document *<br>----- | 1-14 | |
| A | EP 2 508 585 A1 (CHEIL IND INC [KR]) 10 October 2012 (2012-10-10)<br>* claim 9: compound for OLED *<br>* the whole document *<br>----- | 1 | |
| A | CN 106 816 450 B (LG DISPLAY CO LTD) 29 November 2019 (2019-11-29)<br>* claim 5 *<br>* the whole document *<br>----- | 1 | TECHNICAL FIELDS SEARCHED (IPC) |
| A | US 2022/165962 A1 (JI HYE-SU [KR] ET AL) 26 May 2022 (2022-05-26)<br>* compound 36 *<br>* the whole document *<br>----- | 1 | C09K<br>H10K |
| A | EP 3 333 921 A1 (LG DISPLAY CO LTD [KR]) 13 June 2018 (2018-06-13)<br>* claim 5, e.g. EN-162 *<br>* the whole document *<br>----- | 1 | |
| A | WO 2017/095157 A1 (HEESUNG MAT LTD [KR]) 8 June 2017 (2017-06-08)<br>* claim 5, compound 17, compound 42 *<br>* the whole document *<br>----- | 1 | |

The present search report has been drawn up for all claims

1

| Place of search | Date of completion of the search | Examiner |
|---|---|---|
| The Hague | 20 February 2026 | Ziegler, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 21 4112

This annex lists the patent family members relating to the patent documents cited in the above-mentioned European search report. The members are as contained in the European Patent Office EDP file on
The European Patent Office is in no way liable for these particulars which are merely given for the purpose of information.

20-02-2026

| Patent document cited in search report | Publication date | Patent family member(s) | Publication date |
|---|---|---|---|
| KR 20190053579 A | 20-05-2019 | NONE | |
| US 2019263834 A1 | 29-08-2019 | CN 110291088 A | 27-09-2019 |
| | | KR 20180059286 A | 04-06-2018 |
| | | TW 201829404 A | 16-08-2018 |
| | | US 2019263834 A1 | 29-08-2019 |
| | | WO 2018097648 A1 | 31-05-2018 |
| EP 2508585 A1 | 10-10-2012 | EP 2508585 A1 | 10-10-2012 |
| | | JP 2014508130 A | 03-04-2014 |
| | | KR 20120078302 A | 10-07-2012 |
| | | US 2012280613 A1 | 08-11-2012 |
| | | WO 2012091225 A1 | 05-07-2012 |
| CN 106816450 B | 29-11-2019 | CN 106816450 A | 09-06-2017 |
| | | EP 3174124 A1 | 31-05-2017 |
| | | EP 4037001 A1 | 03-08-2022 |
| | | KR 20170062629 A | 08-06-2017 |
| | | KR 20220158216 A | 30-11-2022 |
| | | KR 20230038449 A | 20-03-2023 |
| | | KR 20230113709 A | 01-08-2023 |
| | | US 2017155055 A1 | 01-06-2017 |
| US 2022165962 A1 | 26-05-2022 | CN 113710662 A | 26-11-2021 |
| | | KR 20210017039 A | 17-02-2021 |
| | | TW 202110820 A | 16-03-2021 |
| | | US 2022165962 A1 | 26-05-2022 |
| | | WO 2021025356 A1 | 11-02-2021 |
| EP 3333921 A1 | 13-06-2018 | CN 108218858 A | 29-06-2018 |
| | | EP 3333921 A1 | 13-06-2018 |
| | | KR 20180067321 A | 20-06-2018 |
| | | US 2018166647 A1 | 14-06-2018 |
| WO 2017095157 A1 | 08-06-2017 | CN 108290865 A | 17-07-2018 |
| | | KR 20170064379 A | 09-06-2017 |
| | | TW 201730180 A | 01-09-2017 |
| | | US 2018366652 A1 | 20-12-2018 |
| | | WO 2017095157 A1 | 08-06-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020170093270